# EUROPEAN PATENT SPECIFICATION

(11) **EP 2 029 134 B1**
(45) Date of publication and mention of the grant of the patent: **27.07.2011**
(21) Application number: 07729956.8
(22) Date of filing: 06.06.2007
(51) Int. Cl.: A61K 31/222, A61K 9/16, A61P 13/10

(54) **STABILIZED PHARMACEUTICAL COMPOSITIONS COMPRISING FESOTERODINE**
STABILISIERTE PHARMAZEUTISCHE ZUSAMMENSETZUNG MIT FESOTERODIN
COMPOSITIONS PHARMACEUTIQUES STABILISÉES CONTENANT DE LA FÉSOTÉRODINE

(30) Priority: 09.06.2006 EP 06011941; 09.06.2006 EP 06011942; 09.06.2006 EP 06011943
(43) Date of publication of application: 04.03.2009
(73) Proprietor: UCB Pharma GmbH, 40789 Monheim (DE)
(72) Inventor: ARTH, Dr.Christoph, 40593 Düsseldorf (DE); MIKA, Dr. Hans-Jürgen, 53229 Bonn (DE); KOMENDA, Dr. Michael, 51147 Köln (DE); LINDNER, Dr. Hans, 13465 Berlin (DE); BICANE, Dr. Fatima, 51503 Rösrath (DE); PAULUS, Dr. Kerstin, 40882 Ratingen (DE); IRNGARTIGNER, Dr. Meike, 50226 Frechen (DE)
(74) Representative: Siegert, Georg
(86) International application number: PCT/EP2007/055582
(87) International publication number: WO 2007/141298

(56) References cited:
- EP-A- 0 957 073
- WO-A-03/099268
- WO-A1-01/35957
- WO-A1-99/44604

## Description

### Field of the Invention

The present invention generally relates to a pharmaceutical composition comprising fesoterodine or a pharmaceutically acceptable salt or solvate thereof and to a method for its preparation.

### Background

Fesoterodine is the International Non-proprietary Name (INN) of a compound of formula (I) which can be chemically described as 2-[(1R)-3-(diisopropylamino)-1-phenylpropyl]-4-(hydroxymethyl)phenyl isobutyrate.

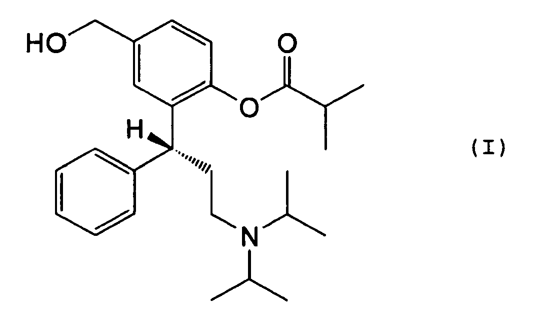

Fesoterodine is an innovative drug for the treatment of overactive bladder, urinary incontinence and other dysfunctions of the urinary tract. It is disclosed, inter alia, in EP 1077912 B1, pertaining to novel derivatives of 3,3-diphenylpropylamines. EP 1230209 B1 (corresponding to WO 01/35957) discloses stable salts of said novel derivatives of 3,3-diphenylpropylamines, including fesoterodine hydrogen fumarate.

WO 03/099268 discloses a combination of a specific opioid and another drug for the treatment of urinary urgency and urinary incontinence. Fesoterodine is mentioned as one example of the drug to be combined with the opioid.

WO 99/44604 discloses a lyophilized pharmaceutical composition containing sodium [[3-(2-amino-1,2-dioxoethyl)-2-ethyl-1-(phenylmethyl)-1H-indol-4-yl]oxy]acetate, a solubilizer and a stabilizer. The stabilizer is at least one compound selected from the group consisting of mannitol, xylitol, sorbitol, glucose, fructose, lactose and maltose.

Overactive bladder (OAB) is an extremely common disorder, affecting 17% of the adult population in major European countries. OAB can occur at any age and in either gender, although its prevalence is higher in geriatric and female populations.

OAB is a bladder function disorder resulting in symptoms of urgency, with or without urge incontinence, and usually includes increased urinary frequency and nocturia. The disorder is due to spastic contractions of the detrusor muscle of the bladder, resulting in sustained high bladder pressure and the urgent need to urinate. This can be caused by several reasons, such as traumatic or toxic nerve damage (e.g., abdominal trauma, pelvic trauma or surgery, bladder stones, adverse effects of drugs, neurological diseases (e.g., spinal cord lesions, multiple sclerosis, Parkinson's disease, excessive neurotransmitter release in the bladder) or myogenic instability (e.g., bladder hypertrophy caused by outlet obstruction or urinary tract infection).

In some cases, OAB can be managed without pharmacotherapy, using exercise, pessaries, implants, biofeedback or behavioral therapy. But in most cases, pharmacotherapy is the better option. Antimuscarinic agents have been found to be particularly effective for treating OAB. During normal micturition, acetylcholine released from postganglionic parasympathetic neurons acts on the muscarinic receptors of the detrusor smooth muscle in the bladder to stimulate contractions. Antimuscarinic agents interfere with this action, thus reducing detrusor contractions. However, despite the availability of different antimuscarinic drugs, physicians and patients remain dissatisfied with current treatments due to adverse events and/or insufficient efficacy.

Therefore, new agents with improved safety and efficacy are needed for a more effective treatment of OAB.

Fesoterodine is known in the art for its potency in treating urinary incontinence. However, fesoterodine may exhibit substantial degradation under stress conditions, e.g., in a humid environment and at increased temperature. It is believed that hydrolyzation and oxidation are among the major mechanisms resulting in degradation. Therefore, it would be desirable to develop new pharmaceutical compositions comprising fesoterodine that are more stable against degradation over an extended period of time even under stress conditions. To that end, it has now been found, surprisingly, that some pharmaceutical excipients are able to significantly slow down the degradation of fesoterodine under stress conditions.

### Summary

The present invention provides a pharmaceutical composition comprising fesoterodine or a pharmaceutically acceptable salt or solvate thereof, and a pharmaceutically acceptable stabilizer. Suitable stabilizers can be selected from the group consisting of xylitol, sorbitol, polydextrose, isomalt, dextrose and combinations thereof. Most preferred is xylitol. Another preferred stabilizer is sorbitol. Another preferred stabilizer is polydextrose.

The pharmaceutical composition of the present invention is preferably suitable for oral administration. Suitable and preferred oral dosage forms of the pharmaceutical compositions according to the present invention are tablets, granulates and coated tablets. Capsules, lozenges and other solid oral administration forms are likewise included in the scope of the present invention.

Also disclosed is a pharmaceutical composition as defined above comprising fesoterodine or a pharmaceutically acceptable salt or solvate thereof, a pharmaceutically acceptable stabilizer as defined above, and at least one further excipient, preferably at least one type of hydroxypropyl methylcellulose.

In yet another aspect the invention relates to a pharmaceutical composition for the oral administration of fesoterodine or a pharmaceutically acceptable salt or solvate thereof that may be obtained by granulating fesoterodine with a suitable excipient, preferably a pharmaceutically acceptable stabilizer as chosen from among xylitol, sorbitol, polydextrose, isomalt, and dextrose, and most preferably xylitol. The granulate then may be mixed with at least one further excipient, preferably at least one type of hydroxypropyl methylcellulose, and optionally other excipients.

In another aspect, disclosed is a pharmaceutical composition comprising fesoterodine or a pharmaceutically acceptable salt or solvate thereof, wherein the pharmaceutical composition can be solid, and may be suitable for oral administration. Fesoterodine, or a salt thereof, can be embedded in a gel matrix formed by at least one type of a water swellable sustained release agent, such as a cellulose ester or ether, and preferably formed by at least one type of hydroxypropyl methylcellulose (hypromellose) and, optionally, further excipients. More preferably, fesoterodine and a stabilizer may be embedded in a gel matrix formed by at least one type of hydroxypropyl methylcellulose (hypromellose) and, optionally, further excipients.

In certain embodiments, the fesoterodine salt can be a salt of a polybasic acid, preferably with an auto pH in water in the range of about 3-5 (measured in water at 25°C at a concentration of 1 wt%). Examples may be chosen from the group of polybasic mineral acids, such as e.g. sulfuric acid and phosphoric acid, or of polybasic organic acids. Preferred examples are salts of di- or tricarboxylic acids such as fesoterodine maleate, fesoterodine oxalate, fesoterodine citrate, fesoterodine phthalate, fesoterodine fumarate, fesoterodine succinate, fesoterodine tartrate, fesoterodine malonate, fesoterodine malate etc. In particular embodiments, the fesoterodine salt may be a partially hydrogenated di- or tricarboxylic acid salt, particularly a salt with an auto pH of 3-5, particularly between 3 and 4, more preferably between 3.25 and 3.75, such as hydrogen fumarate or hydrogen maleate. A particularly preferred salt is fesoterodine hydrogen fumarate.

In yet another aspect, disclosed is a pharmaceutical composition as described above comprising fesoterodine fumarate as a pharmaceutically acceptable salt, and preferably, fesoterodine hydrogen fumarate.

In another aspect, disclosed is a pharmaceutical composition as described above comprising fesoterodine hydrogen fumarate as a pharmaceutically acceptable salt in an amount of about 0.5-28 or 0.5-20 mg, preferably about 1-16 mg, more preferably about 1-12 mg even more preferably about 1-8 mg, and particularly preferably about 2, 4 or 8 mg per dosage unit (based on the content of fesoterodine or its salt, e.g., fesoterodine hydrogen fumarate).

The pharmaceutical compositions of the present invention is used in a method of treating patients suffering from overactive bladder and having symptoms such as urinary incontinence, specifically urinary urge incontinence, imperative urinary urge, and/or increased urinary frequency by administering a therapeutically effective amount of any of the compositions as described herein. In particular, a unit dosage form of the fesoterodine compositions described herein is administered. A unit dosage form may contain between about 0.5-28 or 0.5-20 mg fesoterodine, preferably about 1-16 mg, more preferably about 1-12 mg, even more preferably about 1-8 mg, and particularly preferably about 2, 4 or 8 mg per dosage unit (based on the content of fesoterodine or its salt, e.g., fesoterodine hydrogen fumarate). The unit dosage form can be administered once-daily to a patient or in some cases, more than once daily to a patient as may be appropriate.

Moreover, this application discloses a method for the production of a pharmaceutical composition as described above comprising producing a mixture of fesoterodine or a pharmaceutically acceptable salt thereof and a pharmaceutically acceptable stabilizer as defined above.

These components can then be mixed with at least one type of hydroxypropyl methylcellulose, and optionally other excipients. Optionally, the resultant composition may be pressed into tablets and coated.

One preferred method for the production of a pharmaceutical composition containing fesoterodine comprises granulating a composition containing fesoterodine or a pharmaceutically acceptable salt thereof and a pharmaceutically acceptable stabilizer as defined above and then mixing the granulate thus formed with at least one type of hydroxypropyl methylcellulose, and optionally other excipients. The resultant composition may then be pressed into tablets and coated.

The granulation process may be performed in a dry granulation procedure, without the addition of liquid or, preferably, in the presence of a liquid, such as water ("wet granulation"). In wet granulation, for example, fesoterodine or a pharmaceutically acceptable salt thereof and a stabilizer as defined above can be mixed in the presence of water. The granulate can then be dried. This dried granulate may then be mixed with at least one further excipient, preferably at least one type of hydroxypropyl methylcellulose, and optionally other excipients.

It has been surprisingly found that wet granulation can be accomplished without increasing the degradation of fesoterodine due to hydrolyzation of the ester bond. For the same reason, it was even more surprising that fesoterodine is more stable in a composition that is produced in the presence of water, e.g., by wet granulation, than in a composition that is produced by dry granulation (see, e.g., Table 9) or by dry mixing and compressing the excipients (see Table 8).

### Brief description of the Figures

Figure 1 shows the in vitro dissolution profiles of fesoterodine-containing pharmaceutical compositions based on a 4mg tablet
Figure 2 shows the in vitro dissolution profiles of fesoterodine-containing pharmaceutical compositions based on an 8 mg tablet.

### Detailed Description

In the most general aspect of this application, a pharmaceutical composition is provided comprising fesoterodine or a pharmaceutically acceptable salt thereof, and a pharmaceutically acceptable stabilizer.

### Fesoterodine

Unless the context indicates otherwise, the term "Fesoterodine" as used herein includes pharmaceutically acceptable solvates of 2-[(1R)-3-(diisopropylamino)-1-phenylpropyl]-4-(hydroxymethyl)phenyl isobutyrate (formula I), particularly hydrates of fesoterodine. "Fesoterodine" also includes pharmaceutically acceptable salts of 2-[(1R)-3-(diisopropylamino)-1-phenylpropyl]-4-(hydroxymethyl)phenyl isobutyrate (formula I), particularly the hydrogen fumarate salt, as well as the free base.

Throughout this application, amounts indicated relate to fesoterodine in the form which is used, i.e., either the free base or the salt.

Fesoterodine is an ester which is susceptible to hydrolyzation after administration *in vivo* as well as during storage under stress conditions to give a main product (2-[(1*R*)-3-(diisopropylamino)-1-phenylpropyl]-4-(hydroxymethyl)phenol) (formula II), which is referred to herein as the "Active Metabolite" and corresponds to the following structure:

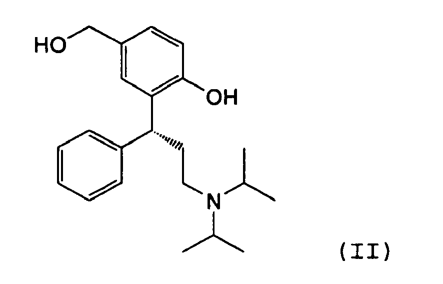

Throughout this application the term "hydrolyzation product" refers to the Active Metabolite of formula II. Other degradation products also may result from the metabolism and/or degradation of fesoterodine. As used herein, "total degradation products" will include, at least in part, hydrolyzation product.

The content of undegraded fesoterodine, as well as the relative amounts of hydrolyzation product and total degradation products given, can be determined via HPLC by the area-% method. In this area-% method the ratio of undegraded fesoterodine is determined by comparing the area of the respective HPLC peak with the total area of all signals in the HPLC profile that can be related to fesoterodine and its hydrolyzation and/or degradation products.

### Stabilizer

A "pharmaceutically acceptable" stabilizer is a stabilizer which is not biologically or otherwise undesirable, i.e., the stabilizer can be administered to an individual without causing significant undesirable biological effects or interacting in a deleterious manner with any of the components of the composition in which it is contained.

"Stabilizer," as the term is used herein, means a substance, particularly a pharmaceutically acceptable excipient, which inhibits, prevents, slows down, or reduces the degradation of fesoterodine as compared to fesoterodine in the absence of the substance under certain conditions. As used herein, the term "stabilizer" also embraces a combination of two or more of the stabilizers according to the invention. By way of example, a combination of xylitol and sorbitol, or a combination of xylitol, sorbitol and polydextrose, or a combination of xylitol and polydextrose may be mentioned.

Stabilizers according to the present invention are chosen from among xylitol, sorbitol, polydextrose, isomalt, and dextrose. Xylitol is one preferred stabilizer. These substances are capable of inhibiting, preventing, slowing down, or reducing the hydrolyzation of fesoterodine into active metabolite of formula (II), and thus act as stabilizers. Thus, the invention relates to pharmaceutical compositions comprising fesoterodine and a stabilizer selected from xylitol, sorbitol, polydextrose, isomalt, and dextrose, and most preferably xylitol, wherein the fesoterodine is protected against hydrolyzation under various stress conditions (see, e.g., Table 6 and Table 7).

Also preferred as stabilizers are those substances that are capable of reducing the destabilizing effect of certain other excipients, such as lactose, on fesoterodine.

### Exemplary pharmaceutical compositions

Exemplary, non-exhaustive examples of various stabilized compositions may be described as follows.

A pharmaceutical composition comprising fesoterodine and a stabilizer as defined above wherein fesoterodine is stable against degradation at about 40°C, 75% r.H. in an open vial such that after about 12 weeks at about 40°C, 75% r.H. in open vial the pharmaceutical composition contains at least about 85%, 86%, 87%, 88%, 89% or about 90% fesoterodine. More preferably, such a pharmaceutical composition contains at least about 89% of fesoterodine after storage under such conditions.

A pharmaceutical composition comprising fesoterodine and a stabilizer as defined above, wherein the fesoterodine is stable against degradation at about 40°C, 75% r.H. in an open vial such that after about 6 weeks at about 40°C, 75% r.H. in open vial the pharmaceutical composition contains no more than about 7%, 6%, 5%, 4.9%, 4.8%, 4.7%, 4.6%, 4.5%, or 4.4% of active metabolite of formula (II).

A pharmaceutical composition comprising fesoterodine and a stabilizer as defined above, wherein the fesoterodine is stable against degradation at about 40°C, 75% r.H. in a closed vial such that after about 6 weeks at about 40°C, 75% r.H. in closed vial the pharmaceutical composition contains no more than about 2.5%, 2.2%, 2.1%, 2%, 1.9%, 1.8%, 1.7%, 1.6%, 1.5%, 1.4%, 1.3%, 1.2%, 1.1%, or 1% of active metabolite of formula (II). More preferably, such a pharmaceutical composition contains no more than about 1.09% of active metabolite of formula (II) after storage under such conditions.

A pharmaceutical composition comprising fesoterodine and a stabilizer as defined above wherein fesoterodine is stable against degradation at about 25°C, 60% r.H. in a closed vial such that after about 6 weeks at about 25°C, 60% r.H. in closed vial the pharmaceutical composition contains no more than about 1%, 0.9%, 0.8%, 0.7%, 0.6%, 0.5%, 0.4%, 0.3%, 0.2%, or 0.1% of active metabolite. More preferably, such a pharmaceutical composition contains no more than about 0.18% of active metabolite of formula (II) after storage under such conditions.

In preferred embodiments, the stabilizer used in the pharmaceutical composition comprising fesoterodine can be polydextrose, sorbitol, or particularly preferably xylitol.

### Granulates

As mentioned above, the pharmaceutical composition of the present invention may be in the form of a granulate. The, granulate may optionally contain at least one further excipient. Whereas fesoterodine itself was found to have degraded after about 12 weeks at about 40°C, 75% relative humidity (r.H.) in open vials to the extent that only about 50% of the original fesoterodine remained in undegraded form, a granulate of fesoterodine and xylitol was found to have about 93% of the original amount of fesoterodine remaining in undegraded form after about 12 weeks under the same conditions.

Thus, exemplary, non-exhaustive examples of fesoterodine granulates are provided.

A granulate of fesoterodine and a stabilizer as defined above wherein the fesoterodine in the granulate is stable against degradation at about 40°C, 75% r.H. in an open vial such that after about 6 weeks at about 40°C, 75% r.H. in open vial the granulate contains no more than about 2%, 1.5%, 1%, 0.9%, 0.8%, 0.7%, 0.6%, or 0.5% of active metabolite of formula (II) by weight. More preferably, such a granulate contains no more than about 0.58% of active metabolite of formula (II) after storage under such conditions.

A granulate of fesoterodine and a stabilizer as defined above wherein the fesoterodine in the granulate is stable against degradation at about 40°C, 75% r.H. in a closed vial such that after about 6 weeks at about 40°C, 75% r.H. in closed vial the granulate contains no more than about 2%, 1.5%, 1%, 0.9%, 0.8%, 0.7%, 0.6%, 0.5%, 0.4%, 0.3%, or 0.2% of active metabolite of formula (II). More preferably, such a granulate contains no more than about 0.23% of active metabolite of formula (II) after storage under such conditions.

A granulate of fesoterodine and a stabilizer as defined above wherein the fesoterodine in the granulate is stable against degradation at about 25°C, 60% r.H. in a closed vial such that after about 6 weeks at 25°C, 60% r.H. in closed vial the granulate contains no more than about 1%, 0.9%, 0.8%, 0.7%, 0.6%, 0.5%, 0.4%, 0.3%, 0.2%, or 0.1% of active metabolite of formula (II). More preferably, such a granulate contains no more than about 0.36%, even more preferably no more than about 0.12% of active metabolite of formula (II) after storage under such conditions.

In preferred embodiments, the granulate includes polydextrose, sorbitol, or particularly preferably xylitol as the stabilizer.

Further provided are granulates of fesoterodine formed by a granulation process, preferably wet granulation, with a suitable excipient, such as a stabilizer as defined above.

A granulate produced from such a granulation step preferably has a fesoterodine/stabilizer ratio of about 1-30% [w/w], more preferably about 1-20% [w/w], more preferably about 3-15% [w/w], and even more preferable about 5-10% [w/w]. In a particularly preferred embodiment, a granulate includes a fesoterodine/xylitol or fesoterodine/sorbitol ratio of about 1-30% [w/w], preferably about 1-20% [w/w], more preferably about 3-15% [w/w], and even more preferably about 5-10% [w/w].

The average size of the granulate can be controlled by usual techniques such as sieving or milling, and typically may be below about 4 mm, preferably below about 2 mm, more preferably below about 1 mm, and even more preferably below about 0.75 mm, e.g. about 0.5 mm.

Fesoterodine granulates as described herein, particularly if produced in a wet granulation process, are surprisingly stable under humid stress conditions. The granulates can be further processed and incorporated into pharmaceutical compositions that are also surprisingly stable.

It has been further surprisingly found that granulating fesoterodine with either xylitol or sorbitol provides for enhanced stability during the granulation process as compared to granulating fesoterodine with either mannitol or maltitol. When fesoterodine was granulated separately with these four sugar alcohols and tested for the amount of hydrolyzation or total degradation that occurred during granulation, it was found that granulating with xylitol or sorbitol resulted in less degradation products than granulating with mannitol or maltitol. Granulating with xylitol or sorbitol led to the formation of about 0.06% to about 0.07% of hydrolyzation products and total degradation products, while granulating with mannitol or maltitol led to the formation of about 0.42% to about 0.73% of hydrolyzation products and total degradation products. (See Table 7).

The surprisingly superior results observed for granulation with xylitol or sorbitol were also observed when fesoterodine granulates including xylitol or sorbitol were used to prepare pharmaceutical compositions. Pharmaceutical compositions in tablet form that were prepared with granulates of fesoterodine that included xylitol or sorbitol exhibited far less hydrolyzation products and total degradation products (about 0.06% to 0.11%) than tablets prepared with fesoterodine granulates containing mannitol or maltitol (about 1.1% to 1.7%). (See Table 8).

The difference between sorbitol and mannitol is especially surprising since these two sugar alcohols are isomers.

In another embodiment the present invention provides the use of granulates comprising fesoterodine and a stabilizer, as described herein, for the treatment of urinary incontinence, specifically urinary urge incontinence, urinary urgency and/or urinary frequency and the preparation of a medicament for treating such conditions. Preferably, the granulates which are used for preparing the medicament (such as, e.g., a tablet) comprise a sugar, sugar alcohol, polyol, or derivative thereof (such as, e.g., xylitol, sorbitol, polydextrose, isomalt, or dextrose). The medicament may be in the form of, e.g., a granulate, a capsule, a tablet or a coated tablet.

### Sustained-release formulations

In yet a further embodiment, provided is a solid pharmaceutical composition for the oral administration of fesoterodine or a pharmaceutically acceptable salt or solvate thereof, preferably fesoterodine hydrogen fumarate, that can be obtained by mixing the granulate described herein with at least one further excipient, preferably with at least one type of sustained release-agents, such as hydroxypropyl methylcellulose (HPMC), and optionally other excipients.

In another aspect, the active ingredient (Fesoterodine or a pharmaceutically acceptable salt thereof) is embedded in a gel matrix formed by a sustained release agent as described hereinafter, preferably by a gel matrix formed by at least one type of a water swellable sustained release agent, such as a cellulose ester or ether, and preferably formed by at least one type of HPMC.

More preferably, a granulate comprising the active ingredient (Fesoterodine or a pharmaceutically acceptable salt thereof) and a stabilizer as defined above , are embedded in a gel matrix formed by at least one type of HPMC. Such a formulation comprising fesoterodine, a stabilizer and a gel matrix formed by at least one type of HPMC is preferably designed to release fesoterodine over an extended period of time, preferably to allow for once-daily oral administration.

Suitably, such a once-daily formulation contains at least about 20% HPMC by weight of the total weight of the formulation, and more preferably at least about 25% (w/w), such as between 25% and 65%, and even more preferably at least about 30% (w/w), such as between 30% and 65%, and particularly preferably at least about 35% (w/w) such as between 35% and 55% HPMC.

The particularly preferred formulations of the present disclosure, such as for examples the formulations "A", "B", "C", "D", "E", "F", "G" and "H" given in tables 1 and 2 of the Experimental part of this application, were shown in clinical studies and/or in bioequivalence studies to be effective for the once-daily administration in man. They all exhibit a particular fesoterodine release profile in in-vitro dissolution assays. These formulations and other formulations showing the respective dissolution profiles form another embodiment of the present disclosure.

Particularly preferable pharmaceutical compositions comprising fesoterodine show a cumulated fesoterodine release (in weight percent based on the theoretical amount of fesoterodine in the formulation) in an in vitro dissolution assay according to USP 711 (in phosphate buffer pH 6.8, 37°C, at 75 rpm) as follows:
● about 5 to about 30%, preferably about 6 to about 26% fesoterodine release after 1 hour,
● about 15% to about 40%, preferably about 18% to about 38% fesoterodine release after 2 hours,
● about 35% to about 65%, preferably about 36% to about 56% fesoterodine release after 4 hours, and
● at least about 75%, preferably at least about 80% fesoterodine release after 16 hours.

The gel matrix for releasing fesoterodine is preferably formed by a sustained release agent, particularly by one that is swellable in contact with water, in an amount of about 20-80% [w/w], preferably about 25-65%, more preferably 30-65%, and even more preferably about 35-55% [w/w] based on the total composition.

The matrix may optionally contain further ingredients. In particular, a filler such as lactose and/or microcrystalline cellulose also may be embedded in the matrix.

In another embodiment, a solid fesoterodine composition comprises at least two types of hydroxypropyl methylcellulose (HPMC). The two types of HPMC may be chemically identical but differ in their viscosity, when dissolved in water under standard conditions. Alternatively, two types of HPMC which differ chemically may be used.

A particularly advantageous composition can be obtained when one type of HPMC has a nominal viscosity of about 100,000 mPa·s (i.e., 100,000 ± 20,000 mPa·s), and the other has a nominal viscosity of about 4,000 mPa·s (i.e., 4,000 ± 1000 mPa·s) when dissolved in water at about 22°C in a concentration of about 2% by weight.

In one preferred embodiment, the ratio of fesoterodine or the pharmaceutically active salt or solvate thereof and HPMC is between about 1:80 and about 1:5 [w/w]. Even more preferred are weight ratios of between about 1:70 and about 1:10 [w/w] and even more preferably between about 1:40 and about 1:15 [w/w].

Another preferred embodiment is a solid pharmaceutical composition that comprises about 0.25 to about 10% [w/w] fesoterodine or its pharmaceutically acceptable salt or solvate. Most preferred compositions include fesoterodine in an amount between about 0.5 and about 4% [w/w]. A preferred composition further may contain one or more additional excipient(s), such as one or more filler(s), binder(s) and/or lubricant(s), and among them lactose, microcrystalline cellulose, talc and glycerol dibehenate are particularly preferred.

### Components of the pharmaceutical composition

The compositions are preferably formulated in a unit dosage form. Each unit dosage form can contain from about 0.5 to 20 mg, preferably about 1-8 mg, and more preferably about 2, 4, or 8 mg of fesoterodine or a pharmaceutically acceptable salt thereof, such as, e.g., the hydrogen fumarate salt. The term "unit dosage form" refers to physically discrete units suitable as unitary dosages for human beings or other mammals, each unit containing a predetermined quantity of fesoterodine or its salt calculated to produce the desired therapeutic effect, in addition with suitable pharmaceutical excipients. Most preferred are solid administration forms (such as tablets, coated tablets, granulates and capsules) that only require a once-daily administration to the patient to achieve the desired therapeutic effect.

Pharmaceutical excipients that may be present in the compositions described herein include sustained release ("SR") agents, disintegrants, fillers and lubricants. Other excipients can also be included.

### Sustained release ("SR") agents

If the pharmaceutical composition of the present invention is a sustained release formulation, it will usually contain one or more sustained release agents. Suitable sustained release agents are preferably those that swell upon contact with water such as polyvinylpyrrolidone, pregelatinised starch, polymethacrylate, polyvinylacetate, dextranes, starch, cellulose and cellulose ethers and esters like methylcellulose, methylethylcellulose, hydroxyethylcellulose, hydroxypropylmethylcellulose, hydroxypropylcellulose, or carboxymethylcellulose, or mixtures thereof.

Preferred examples of sustained release agents are hydroxypropyl methylcellulose (HPMC) or mixtures of different HPMCs. HPMC may act both as a binder and sustained release agent. HPMC is preferably present in an amount that allows for the formation of a gel matrix from which the active ingredient is gradually released.

In a preferred embodiment, two types of hydroxypropyl methylcellulose of a different viscosity are used. In these mixtures, one HPMC may have a high viscosity and one HPMC may have a low viscosity.

"High viscosity HPMCs" are those having (at 22°C) a nominal viscosity (by Ubbelohde viscometers) of between about 70,000 and about 150,000, and preferably of about 100,000 (i.e., 100,000 ± 20,000) mPa·s when dissolved (about 2% by weight) in water. "low viscosity HPMCs" refers to HPMCs having at room temperature a nominal viscosity of between about 3,000 and about 20,000, and preferably of about 4,000 mPa.s (i.e., 4,000 ± 1,000 mPa·s) when dissolved (about 2% by weight) in water. Preferably the rate of substitution with methoxyl groups of the HPMCs used is between about 15 and about 35%, and particular preferred between about 18 and about 30%, while the rate of substitution with hydroxypropoxy groups is preferably between about 5 and about 14%, and more preferably between about 7 and about 12%. Suitable qualities are found in, for example, METHOCEL® E4M, METHOCEL® K4M, METHOCEL® K15M and METHOCEL® K100M which are obtainable from the Dow Chemical Company.

Particularly preferred brands are METHOCEL® K100M having a nominal viscosity of about 100,000 mPa.s and METHOCEL® K4M having a nominal viscosity of about 4,000 mPa·s. The weight ratios of METHOCEL® K100M and K4M used in the compositions and formulations described herein can be in the range of about 20:1 to about 1:2, and are preferably in the range of about 10:1 to about 1:1.5, and are even more preferably in the range of about 7:1 to about 1:1.3.

In one embodiment, one distinct type of HPMC may be used, such as, e.g., HPMC with a nominal viscosity of between about 50,000 and about 120,000 mPa·s, wherein HPMC with a viscosity of between about 50,000 and about 100,000 mPa·s is preferred.

### Disintegrants

Further, the compositions and formulations described herein can also contain disintegrants, such as pregelatinized starch, sodium starch glycolate, microcrystalline cellulose, carboxymethylcellulose sodium (CMC-Na), cross-linked CMC-Na, polacrilin potassium, low-substituted hydroxypropylcellulose or mixtures thereof. The presence of a disintegrant in compositions and formulations described herein is not necessary, but may be desirable.

### Fillers/Binders

The pharmaceutical compositions described herein can further contain fillers and/or binders such as microcrystalline cellulose, powdered cellulose, compressible sugar, starch (e.g., corn starch or potato starch), pregelatinized starch, fructose, sucrose, dextrose, dextranes, other sugars such as saccharose, siliconized microcrystalline cellulose, calcium hydrogen phosphate, calcium hydrogen phosphate dihydrate, dicalciumphosphate dihydrate, tricalciumphosphate, calcium lactate or mixtures thereof.

Preferably, the excipients include at least one filler selected from microcrystalline cellulose and lactose monohydrate. More preferably, a mixture of lactose and microcrystalline cellulose in a ratio of about 1:1 to about 3:1 [W/W] is used as the excipient. A particularly preferred excipient is MICROCELAC® 100, which is a co-processed mixture of lactose monohydrate and microcrystalline cellulose in a ratio of 3:1, both of pharmacopoeial quality, manufactured by combined spray-drying. Both the filling properties of lactose and the binding capacity of microcrystalline cellulose are synergistically co-processed to one excipient providing improved flow properties and better tabletting performance to the composition.

The compositions described herein also can comprise binders, such as cellulose derivatives (e.g., methylcellulose and sodium carboxymethylcellulose), gelatin, glucose, lactose, sucrose, polyethylene glycol, polymethacrylates, hydroxypropylcellulose, sugar alcohols, pregelatinized starch and sodium alginate. These may be helpful to form granules. Some of the preferred stabilizers such as xylitol and sorbitol also have binder properties.

If binders are used to form granulates, they can preferably be used in a mean particle size of about 1-300µm, more preferably about 1-200µm, and even more preferably about 5-100 µm. Most preferably, the binder particles should be smaller than about 1 mm.

For example, if xylitol is used to form granules, suitable qualities are provided by XYLITAB® 300 or XYLITOL® CM50 (both produced by Xyrofin Oy, Kotka, Finland and commercialized by Danisco), and XYLITOL 90 (produced by Roquette GmbH, Germany)

### Lubricants

The compositions and formulations disclosed herein can also comprise lubricants, antiadherents and/or glidants such as stearic acid, magnesium stearate, calcium stearate, sodium lauryl sulphate, hydrogenated vegetable oil, hydrogenated castor oil, sodium stearyl fumarate, macrogols, glycerol dibehenate, talc, corn starch, silicone dioxide or mixtures thereof.

The preferred lubricants are talc and glycerol dibehenate.

The term "glycerol dibehenate" as used herein shall be considered synonymous with "glyceryl behenate".

### Coatings

Optionally, compositions and formulations described herein, including cores/tablets, can be coated with conventional materials used for film coating, e.g., as described in Pharmaceutical Coating Technology, 1995, edited by Graham Cole. Film coating compositions usually contain the following components: polymer(s), plasticizer(s), colorant(s) / opacifier(s), vehicle(s). Minor quantities of flavours, surfactants and waxes also can be used in the film coating solution or suspension. The majority of the polymers used in film coatings are either cellulose derivatives, such as the cellulose ethers, or acrylic polymers and copolymers. Occasionally encountered are high molecular weight polyethylene glycols, polyvinylpyrrolidone, polyvinyl alcohol and waxy materials.

Typical cellulose ethers are hydroxyethylcellulose, hydroxypropylcellulose, hydroxypropyl methylcellulose and methylcellulose. Acrylic polymers comprise a group of synthetic polymers with diverse functionalities. Some of them can be further modified to enhance swelling and permeability by the incorporation of materials such as water soluble cellulose ethers and starches in order to ensure complete disintegration/dissolution of the film.

The commonly used plasticizers can be categorized into three groups: polyols (glycerol, propylene glycol, macrogols), organic esters (phthalate esters, dibutyl sebacetate, citrate esters, triacetin), oils/glycerides (castor oil, acetylated monoglycerides, fractionated coconut oil).

Colorants/opacifiers are classified into several groups: organic dyes and their lakes, inorganic colors, natural colors. Different materials from each group can also be combined in defined ratios.

One suitable composition of a coating suspension (calculated on dry material) comprises:
● about 1-99% by weight of polymer, preferably about 1-95% of polymer,
● about 1-50% by weight of plasticizer, preferably about 1-40% of plasticizer,
● about 0.1-20% of colorant/opacifier, preferably about 0.1-10% of colorant/opacifier.

Film coats may be prepared from ready-to-make preparations which are available on the market. One preferred film-coat material is OPADRY®, particularly OPADRY® blue, which is a mixture of 6 components, i.e., polyvinyl alcohol (film forming agent), PEG (plasticizer), lecithin (emollient), talc (lubricant), titanium dioxide (white pigment), indigo carmine aluminium lake (dye). Depending on the desired opacity, the preferred amount of the coating is about 4-6% w/w of the tablet, preferably about 4.5%.

A film coating dispersion or suspension can be prepared by using different solvents (water, alcohols, ketones, esters, chlorinated hydrocarbons), but water is preferred.

While a coating may in principle be used to modify the release of the active ingredient from a formulation, the coating of the pharmaceutical formulation of the present invention does preferably not or not significantly modify the release profile of fesoterodine. Preferably, the coating is applied to the fesoterodine formulation for patient's convenience only, i.e. to improve the handling and the appearance of the final tablet.

### Preferred quantities of ingredients

Preferred exemplary, non-exhaustive examples of solid pharmaceutical compositions will now be described. All percentages are weight based [w/w], relating to the total weight of the composition, unless indicated otherwise.

One embodiment relates to a pharmaceutical composition that comprises:
- About 0.3-5.0% [w/w] of fesoterodine or a salt thereof, preferably fesoterodine hydrogen fumarate.
- About 5-25% [w/w] of stabilizer, e.g. xylitol, sorbitol, polydextrose, isomalt, or dextrose. The stabilizer preferably has a mean particle size of about 0.001-0.30 mm).
- About 20-40% [w/w] of fillers and/or binders, such as lactose monohydrate and microcrystalline cellulose.
- About 20-65% [w/w] hydroxypropyl methylcellulose, and preferably about 25-65%, more preferably 30-65%, and even more preferably about 35-55% [w/w] hydroxypropyl methylcellulose.
- About 1-10% [w/w] lubricants, such as glycerol dibehenate and/or talc.

More preferable is a pharmaceutical composition that comprises:
- about 0.3-5.0% [w/w] of fesoterodine or a salt thereof, preferably fesoterodine hydrogen fumarate;
- about 5-25% [w/w] of stabilizer, e.g. xylitol, sorbitol, polydextrose, isomalt, or dextrose (preferably having a mean particle size of 0.001-0.30 mm);
- about 20-40% [w/w] of a mixture comprising about 45-80%, preferably about 75% (w/w) lactose monohydrate and about 55-20%, preferably about 25% (w/w) microcrystalline cellulose;
- about 20-65%, preferably about 25-65%, more preferably about 30-65%, and even more preferably about 35-55% [w/w] hydroxypropyl methylcellulose;
- about 1-10% [w/w] lubricants, such as glycerol dibehenate and/or talc.

In another specific embodiment, a pharmaceutical composition comprises
- about 0.5-4.0% [w/w] of fesoterodine hydrogen fumarate;
- about 5-25% [w/w] of stabilizer, e.g. xylitol, sorbitol, polydextrose, isomalt, or dextrose (preferably having a mean particle size of about 0.001-0.30 mm);
- about 20-40% [w/w] of a mixture comprising about 45-80%, preferably about 75% (w/w) lactose monohydrate and about 55-20%, preferably about 25% (w/w) microcrystalline cellulose;
- about 15-55%, and preferably about 15-40% [w/w] high viscosity hydroxypropyl methylcellulose;
- about 5-30%, and preferably about 5-25% [w/w] low viscosity hydroxypropyl methylcellulose;
- about 1-5% [w/w] glycerol dibehenate; and
- about 1-5% [w/w] talc.

A coating may optionally be applied to such a composition. One preferred material for film-coating is OPADRY®, but other coatings are known.

Yet another embodiment relates to a pharmaceutical composition that comprises:
● about 0.1-10%, preferably about 0.2-7%, more preferably between 0.3 and 5%, and more preferably about 0.5-4.0% fesoterodine or a salt thereof, preferably the hydrogen fumarate salt,
● about 20-65%, preferably about 25-65%, more preferably about 30-65%, and even more preferably about 35-55% HPMC, wherein the HPMC may comprise two or more different types, such as a high-viscosity HPMC (e.g., METHOCEL® K100M) and a low-viscosity HPMC (e.g., METHOCEL® K4M). The ratio of high-viscosity HPMC to low-viscosity HPMC may be about 20:1 to about 1:2 (w/w), and preferably is about 10:1 to about 1:1.5 (w/w), and even more preferably is about 7:1 to 1:1.3 (w/w).
● about 1-45%, preferably about 2-35%, and more preferably about 5-25% of stabilizer, e.g. xylitol, sorbitol, polydextrose, isomalt, or dextrose,
● about 10-70%, preferably about 15-50%, and more preferably about 20-40% a filler, and
● about 0.5-10%, preferably about 1-8%, and more preferably about 2-7% a lubricant.

A particularly preferred solid pharmaceutical composition comprises [w/w]:

| | |
|---|---|
| Fesoterodine hydrogen fumarate | about 0.5-4.0% |
| Xylitol | about 5-25% |
| MICROCELAC® 100 | about 20-40% |
| HPMC (high viscosity) | about 15-40% |
| HPMC (low viscosity) | about 5-25% |
| Glycerol dibehenate | about 1-5% |
| Talc | about 1-5% |

and optionally a pharmaceutically acceptable coating. In this particularly preferred embodiment the preferred mean particle size of xylitol is about 1-300µm, more preferably about 1-200µm, and even more preferably about 5-100 µm. In the most preferred embodiment, all particles should preferably be less than 1 mm. The same general and preferred particle sizes likewise apply to other stabilizers according to the invention.

Another preferred embodiment is a coated tablet comprising a core that further comprises:

| | |
|---|---|
| Fesoterodine hydrogen fumarate | about 4.0 mg |
| Xylitol (preferably with a mean particle size of about 0.001-0.30 mm) | about 32-40 mg |
| MICROCELAC® 100 | about 115-130 mg |
| HPMC having a nominal viscosity of about 100,000 mPa·s when dissolved (about 2% by weight) in water, preferably METHOCEL® K100M | about 65-75 mg |
| HPMC having a nominal viscosity of about 4,000 mPa·s when dissolved (about 2% by weight) in water, preferably METHOCEL® K4M | about 65-75 mg |
| Glycerol dibehenate | about 8-12 mg |
| Talc | about 7-10 mg |

and a coating, preferably OPADRY®, preferably in an amount of between about 10 and 20 mg, and more preferably about 15 mg OPADRY®.

Another preferred embodiment is a coated tablet comprising a core that further comprises:

| | |
|---|---|
| Fesoterodine hydrogen fumarate | about 4.0 mg |
| Xylitol (preferably with a mean particle size of about 0.001-0.30 mm) | about 36.0 mg |
| MICROCELAC® 100 | about 121.5 mg |
| HPMC having a nominal viscosity of about 100,000 mPa·s when dissolved (about 2% by weight) in water, preferably METHOCEL® K100M | about 70.0 mg |
| HPMC having a nominal viscosity of about 4,000 mPa·s when dissolved (about 2% by weight) in water, preferably METHOCEL® K4M | about 70.0 mg |
| Glycerol dibehenate | about 10.0 mg |
| Talc | about 8.5 mg |

and a coating, preferably OPADRY®, preferably in an amount of between about 10 and 20 mg, and more preferably about 15 mg OPADRY®.

The pharmaceutical compositions in addition may contain minor amounts (less than about 3%, and more preferably less than about 1% by weight) of impurities. Moreover, the compositions preferably contain no more than about 5% by weight water which may be used during the manufacturing process.

In yet another embodiment, it is a coated tablet comprising a core comprising:

| | |
|---|---|
| Fesoterodine hydrogen fumarate | about 8.0 mg |
| Xylitol (preferably with a mean particle size of about 0.001-0.20 mm) | about 65-80 mg |
| MICROCELAC® 100 | about 70-85 mg |
| HPMC having a nominal viscosity of about 100,000 mPa·s when dissolved (about 2% by weight) in water, preferably METHOCEL® K100M | about 110-130 mg |
| HPMC having a nominal viscosity of about 4,000 mPa·s when dissolved (about 2% by weight) in water, preferably METHOCEL® K4M | about 20-30 mg |
| Glycerol dibehenate | about 8-12 mg |
| Talc | about 7-10 mg |

and a coating, preferably OPADRY®, preferably in an amount of between about 10 and 20 mg, and more preferably about 15 mg OPADRY®.

In yet another embodiment, it is a coated tablet comprising a core comprising:

| | |
|---|---|
| Fesoterodine hydrogen fumarate | about 8.0 mg |
| Xylitol (preferably with a mean particle size of about 0.001-0.20 mm) | about 72.0 mg |
| MICROCELAC® 100 | about 77.5 mg |
| HPMC having a nominal viscosity of about 100,000 mPa·s when dissolved (about 2% by weight) in water, preferably METHOCEL® K100M | about 120.0 mg |
| HPMC having a nominal viscosity of about 4,000 mPa·s when dissolved (about 2% by weight) in water, preferably METHOCEL® K4M | about 24.0 mg |
| Glycerol dibehenate | about 10.0 mg |
| Talc | about 8.5 mg |

and a coating, preferably OPADRY®, preferably in an amount of between about 10 and 20 mg, and more preferably about 15 mg OPADRY®.

### Preparation Process

The present pharmaceutical compositions can be prepared by known procedures, e.g., compression or granulation. In the preparation of the pharmaceutical compositions, fesoterodine or a pharmaceutically acceptable salt or solvate thereof usually can be mixed with an excipient or mixture of excipients, or diluted by an excipient or mixture of excipients, or enclosed within an excipient or mixture of excipients.

Granulates comprising fesoterodine or a pharmaceutically acceptable salt or solvate thereof can be produced by granulation, for example by dry granulation or, preferably, by wet granulation. Wet granulation is usually performed by adding a liquid, e.g., water, to a mixture of the active ingredient (i.e., fesoterodine or its salts or solvates) and further excipients to form granulates (e.g., a sugar alcohol selected from sorbitol and xylitol) and then granulating the wet mixture.

In one embodiment, wetting a mixture of fesoterodine and sorbitol or xylitol can be performed in conventional granulation equipment, such as a high shear mixer (e.g., Lödige MGT 250) or fluid bed spray dryer (e.g., Glatt GPCG 60/90), by spraying a liquid such as, e.g., ethanol, isopropanol, aqueous solutions of ethanol or isopropanol, or preferably water or an aqueous granulating liquid onto the mixture of fesoterodine and xylitol or sorbitol by conventional pharmaceutical techniques. Wetting also can be performed by direct addition of a liquid, such as water or an aqueous granulating liquid to the above mixture during a mixing operation in a proper mixing device, e.g., a high-shear mixer granulator. The term "aqueous granulating liquid" refers to an aqueous dispersion which contains purified or demineralised water (Ph.Eur.) as a liquid and a solid substance which is dispersed, suspended or dissolved in the liquid.

The mixing of excipients alone or with fesoterodine may be effected in conventional devices used for mixing of powders, e.g., motionless (passive) mixers, fluidized bed, diffusion, biconic diffusion, biconic, turbular, cubic, planetary, Y-, V-shaped or high-shear mixers.

For drying the wet granulate, conventional drying systems such as fluid-bed dryers or drying chambers can be used.

In the processes as described above, compression, in particular to tablets, can be effected using an automatic rotary tablet machine from different manufacturers of equipment for use in the pharmaceutical industry.

Conventional equipment can be used for applying a film coating, such as the Driacoater 1200 coating system or other conventional coating pans used in the pharmaceutical industry.

The process for preparing the pharmaceutical composition can be carried out as a wet or dry granulation process or as a direct compression process.

It has been determined, surprisingly, that fesoterodine is particularly stable in a pharmaceutical composition that is produced in the presence of a liquid, preferably water, and particularly if the pharmaceutical composition comprising fesoterodine is produced via a wet granulation process, particularly preferably if water is used as the liquid in the wet granulation process.

Since fesoterodine contains an ester functional group, it was expected that exposing fesoterodine to water would lead to more hydrolyzation of the ester than not exposing fesoterodine to water. Thus, prior to the Applicants' work, more hydrolyzation was expected from wet granulation processes than from dry granulation processes. Instead, surprisingly, for example, pharmaceutical compositions of fesoterodine and xylitol in the form of tablets that were produced by a process including wet granulation were tested for stability for 6 weeks at 40°C, 75% r.H. in closed vials and were found to contain only about 0.5% hydrolyzation product (i.e., active metabolite) and only about 0.7% total degradation products (see Table 8). In contrast, tablets comprising fesoterodine and xylitol that were produced by direct compression (i.e., a process not including wet granulation), stored under the same conditions, were found to contain about 1.3% hydrolyzation product (i.e., active metabolite) and about 2.1% total degradation products (see Table 8).

Accordingly, another embodiment provided herein is a pharmaceutical composition comprising fesoterodine and xylitol, which pharmaceutical composition contains no more than about 0.5% (and in particular, no more than about 0.48%) hydrolyzation product (i.e., active metabolite) and no more than about 0.7% (and in particular, no more than about 0.68%) total degradation products after storage for 6 weeks at about 40°C, 75% r.H. in closed vials.

Pharmaceutical compositions of fesoterodine and xylitol in the form of tablets that were produced by a process including wet granulation were tested for stability for 6 months at room temperature in closed vials, and were found to contain only about 0.2% hydrolyzation product (i.e., active metabolite) (by weight as compared to fesoterodine) and only about 1.3% total degradation products (see Table 9). In contrast, tablets comprising fesoterodine and xylitol that were produced by dry granulation, stored under the same conditions, were found to contain about 0.8% hydrolyzation product (i.e., active metabolite) and about 1.7% total degradation products (by weight as compared to fesoterodine) (see Table 9).

Accordingly, also provided herein are is a pharmaceutical composition comprising fesoterodine and xylitol, which pharmaceutical composition contains no more than about 0.2% (and in particular, no more than about 0.27%) hydrolyzation product (i.e., active metabolite) and no more than about 1.3% (and in particular, no more than about 1.33%) total degradation products after storage for 6 months at room temperature in closed vials.

Also provided is an improved process for producing a granulate of fesoterodine and a stabilizer as defined above comprising the step of wet granulating fesoterodine and the stabilizer. Similarly, provided is a granulate of fesoterodine and a, stabilizer as defined above obtainable by or produced by a process comprising the step of wet granulating fesoterodine and the stabilizer as defined above. In a preferred embodiment, water is used in wet granulation.

The process of wet granulation may comprise:
(a) providing a mixture of fesoterodine and a stabilizer as defined above;
(b) adding water to the mixture to form a wet mixture; and
(c) granulating the wet mixture.

The ratio of fesoterodine: stabilizer in the mixture of step (a) may be from about 1:1 to about 1:20, and is more preferably from about 1:1 to about 1:10.

In a preferred embodiment, the wet aqueous granulation process comprises:
● providing fesoterodine that is optionally micronized or a pharmaceutically acceptable salt or solvate thereof,
● granulating said fesoterodine or said salt or solvate thereof together with suitable excipients such as, e.g., a stabilizer as defined above using water or a water-based dispersion as granulation liquid to obtain a granulate,
● mixing the above, optionally with HPMC and/or other excipients to give a compression mixture,
● compressing the compression mixture to the desired form, and
● optionally applying a coating.

In yet another embodiment, a pharmaceutical composition comprising fesoterodine or pharmaceutically acceptable salts thereof is produced by:
(a) providing a dry mixture of fesoterodine and a sugar, sugar alcohol, polyol, or derivative thereof preferably selected from xylitol, sorbitol, polydextrose, isomalt, and dextrose;
(b) adding a liquid, preferably water, to the mixture obtained in (a) to form a wet mixture;
(c) granulating the wet mixture;
(d) drying the granulates;
(e) mixing the granulates with at least one other excipient, preferably at least one type of hydroxypropyl methylcellulose to form a mixture of granulates and the at least one other excipient, preferably hydroxypropyl methylcellulose, and optionally adding further excipients;
(f) pressing the mixture of granulates and the at least one other excipient, preferably hydroxypropyl methylcellulose into tablets; and
(g) coating the tablets.

The pharmaceutical compositions described herein in the form of a tablet, or optionally a coated tablet, are surprisingly stable under harsh conditions (40°C, 75% relative humidity). The shelf-life of a preferred composition at room temperature can be as long as 2 years.

### Examples

### 1. Preparation of the granulate by wet granulation

● 6.4 kg of fesoterodine hydrogen fumarate and 57.6 kg of xylitol were weighed separately. If xylitol had agglomerated, it was passed through a sieve (1.5 mm).
● Fesoterodine hydrogen fumarate and xylitol were passed through a sieve (0.8 mm).
● The materials were transferred into a suitable high-shear mixer granulator (e.g., Lödige Diosna V25 or Lödige Diosna P1/6) and mixed for 1 minute.
● 3.6 L of purified water were added to the dry mixture while stirring.
● The mixture was stirred with a chopper for 90 to 120 seconds.
● The mixer was emptied, and the contents were transferred to a sieving machine. The wet granulate was passed through a sieve or a screen (4.0 mm).
● The sieved granulate was dried on trays at 45°C for a minimum of 8 h in a drying chamber/oven until a water content of not more than 0.5%.
● The dried granulate was passed through a sieve (0.5 to 1.0 mm).
● Mixing (speed: 8 rpm) was performed for 5 minutes.

### 2. Preparation of the press mixture

### 2.1. 4 mg SR tablets

● 16.0 kg of the granulate and 48.6 kg of MICROCELAC® 100 were passed through a sieve (0.5 to 1.0 mm) (e.g., FREWITT), transferred into a mixing container and mixed (speed: 8 rpm) for 10 minutes.
● 28.0 kg of hypromellose (e.g., METHOCEL® K100M), 28.0 kg of hypromellose (e.g., METHOCEL® K4M), 4.0 kg of glycerol dibehenate and 3.4 kg of talc were added to the pre-mixture and mixed (speed: 8 rpm) for 1 minute.
● The mixture was passed through a sieve (0.5 to 1.0 mm) (e.g., FREWITT) into a mixing container.
● Mixing (speed: 8 rpm) for 10 to 30 minutes was performed.

### 2.2. 8 mg SR tablets

● 32.0 kg of granulate and 31.0 kg of MICROCELAC® 100 were passed through a sieve (0.5 to 1.0 mm) (e.g., FREWITT), transferred into a mixing container and mixed (speed: 8 rpm) for 10 minutes.
● 48.0 kg of hypromellose (e.g., METHOCEL® K100M), 9.6 kg of hypromellose (e.g., METHOCEL® K4M), 4.0 kg of glycerol dibehenate and 3.4 kg of talc were added to the pre-mixture and mixed (speed: 8 rpm) for 1 minute.
● The mixture was passed through a sieve (0.5 to 1.0 mm) (e.g., FREWITT) into a mixing container.
● Mixing (speed: 8 rpm) for 10 to 30 minutes was performed.

### 3. Tabletting

The finished press mixture was transferred to a rotary tablet machine and compressed to oval biconvex tablets.

### 4. Coating

OPADRY® and purified water were added to a vessel under stirring. The mixture was stirred for at least 1 h. Then the suspension was passed through a sieve of a suitable size (e.g., 300 µm) and transferred into the solution tank of the coating pan (e.g., Driacoater 1200). A film coating was applied on the cores under constant stirring of the suspension until the weight per coated tablet was 335 mg.

### 5. Exemplary Compositions of Sustained Release Compositions

**Table 1: Compositions of fesoterodine 4 mg SR tablets**

| | **EXAMPLES** | | |
|---|---|---|---|
| | A | B | C |
| Tablet Core | | | |
| Fesoterodine hydrogen fumarate | 4.0 | 4.0 | 4.0 |
| Xylitol | 36.0 | 36.0 | 36.0 |
| Lactose monohydrate (75%) / microcryst. cellulose (25%) (e.g. MICROCELAC® 100) | 124.5 | 121.5 | 121.5 |
| Hypromellose (e.q. METHOCEL® K100M) | 70.0 | 70.0 | 70.0 |
| Hypromellose (e.g. METHOCEL® K4M) | 70.0 | 70.0 | 70.0 |
| Glycerol dibehenate | 8.0 | 10.0 | 10.0 |
| Talc | 7.5 | 8.5 | 8.5 |
| Purified water | q.s.* | q.s.* | q.s.* |
| | | | |
| Film-coat | White 10.0 | White 10.0 | Blue 15.0 |
| Purified water | q.s.* | q.s.* | q.s.* |
| | | | |
| Total | 330.0 | 330.0 | 335.0 |

| | | | |
|---|---|---|---|
| q.s. = quantum satis = as much as needed * removed during drying of the wet granulate or during film-coating, to a total residual moisture of approx. 2.5-3.5% Numbers are in milligrams. | | | |

**Table 2: Compositions of fesoterodine 8 mg SR tablets**

| | **EXAMPLES** | | | | |
|---|---|---|---|---|---|
| | D | E | F | G | H |
| Tablet core | | | | | |
| Fesoterodine hydrogen fumarate | 8.0 | 8.0 | 8.0 | 8.0 | 8.0 |
| Xylitol | 72.0 | 72.0 | 72.0 | 72.0 | 72.0 |
| Lactose monohydrate (75%) / microcryst. cellulose (25%) (e.g. MICROCELAC® 100) | 80.5 | 140.0 | 80.5 | 77.5 | 77.5 |
| Hypromellose (e.g. METHOCEL® K100M) | 120.0 | 165.0 | 120.0 | 120.0 | 120.0 |
| Hypromellose (e.g. METHOCEL® K4M) | 24.0 | 33.0 | 24.0 | 24.0 | 24.0 |
| Glycerol dibehenate | 8.0 | 11.0 | 8.0 | 10.0 | 10.0 |
| Talc | 7.5 | 11.0 | 7.5 | 8.5 | 8.5 |
| Purified water | q.s.* | q.s.* | q.s.* | q.s.* | q.s.* |
| | | | | | |
| Film-coat | 15.0 | 15.0 | 10.0 | 10.0 | 15.0 |
| Purified water | q.s.* | q.s.* | q.s.* | q.s.* | q.s.* |
| | | | | | |
| Total | 335.0 | 455.0 | 330.0 | 330.0 | 335.0 |

| | | | | | |
|---|---|---|---|---|---|
| q.s. = quantum satis, as much as needed * removed during drying of the wet granulate or during film-coating, to a total residual moisture of approx. 2.5-3.0% Numbers are in milligrams. | | | | | |

### 6. Stability Tests

### a) Storage of tablets in closed containers

The tablet compositions of Examples A, F, and G containing 4, 8, and 8 mg fesoterodine hydrogen fumarate, respectively, were tested for stability. The results are shown in Table 3 below.

The undegraded fesoterodine in the tablets was measured under the following HPLC conditions and taken as an indication of stability:
● Column: Prontosil Spheribond CN, 5 µm, 250 mm x 4 mm or equivalent
● Component A: Water/trifluoroacetic acid 1000/1 (v/v)
● Component B: Acetonitrile/trifluoroacetic acid 1000/1 (v/v)
● Gradient profile:

| Time (min) | %A | %B |
|---|---|---|
| 0.0 | 75 | 25 |
| 10.0 | 75 | 25 |
| 10.1 | 50 | 50 |
| 19.0 | 50 | 50 |

● Flow rate: 1.2 mL/min.
● Column temperature: 35°C
● Injection volume: 75 µL
● Detection wavelength: 220 nm

The retention time of the active metabolite was about 4.7 min; response factor: 1.5.

Evaluation of the HPLC results was by the area percent method.

The average content of hydrolyzation or degradation product (% by weight) observed after storage at 25°C, 60% r.H. in closed containers (25 mL brown glass bottle sealed with a plastic cap and a paraffin sealing, without any desiccant), for representative compositions is shown in Table 3 below.

**Table 3**

| Time of Storage | Ex. A 4mg %Hydr | Ex. F 8mg %Hydr | Ex. G 8mg %Hydr | Ex. G 8mg %Degr |
|---|---|---|---|---|
| Initial | 0.47 | 0.51 | 0.11 | 0.69 |
| 3 months | 0.50 | 0.58 | 0.16 | 1.05 |
| 6 months | 0.53 | 0.69 | 0.21 | 1.10 |
| 9 months | 0.70 | 0.65 | 0.31 | 1.20 |
| 12 months | 0.88 | 0.75 | 0.39 | 1.37 |
| 18 months | 0.85 | 1.01 | 0.37 | 1.26 |
| 24 months | 0.94 | 1.14 | 0.78 | 1.95 |

| | | | | |
|---|---|---|---|---|
| %Hydr = Hydrolyzation product of fesoterodine (active metabolite) [% by weight] %Degr = Total degradation of fesoterodine [% by weight] | | | | |

### b) Storage in open vials

Fesoterodine hydrogen fumarate, a granulate containing fesoterodine hydrogen fumarate and xylitol in a weight-ratio of 10:90, as well as a composition comprising fesoterodine hydrogen fumarate and xylitol were stored in open vials (25 ml brown glass bottles) for up to 12 weeks at 40°C and 75% r.H. The results are shown in Table 4 below.

The degradation of fesoterodine was measured by HPLC. The following conditions were used to measure degradation products in the stability testing of fesoterodine hydrogen fumarate:
● Column: Polaris C18-Ether, 3 µm, 250 mm x 4.6 mm
● Eluent A: Water/methanesulfonic acid 1000 : 0.5 (v/v)
● Eluent B: Acetonitrile/methanesulfonic acid 1000 : 0.5 (v/v)
● Typical gradient profile:

| Time (min) | %A | %B |
|---|---|---|
| 0.0 | 67 | 33 |
| 16.0 | 38 | 62 |
| 18.0 | 0 | 100 |

● Column temperature: 35°C
● Flow rate: 1.2 mL/min
● Detection wavelength: 220 nm
● Injection volume: 20 µL

The retention time of the active metabolite was about 4.1 min (rrt = 0.50); response factor: 1.4.

### Evaluation of the HPLC results was by the area percent method.

A comparison of the observed average of undegraded fesoterodine of a fesoterodine-containing granulate and a corresponding composition as compared to pure fesoterodine after open storage under stress conditions is set out in Table 4, wherein values are indicated in % of fesoterodine hydrogen fumarate remaining at various times.

**Table 4**

| Storage at 40°C and 75% r.H. | Fesoterodine (pure) | Granulate containing fesoterodine/xylitol 10%/90% (wt/wt) | Composition (Ex.B) |
|---|---|---|---|
| Initial | 98.96% | 99.04% | 99.18% |
| 1 week | 98.56% | 98.83% | not measured |
| 2 weeks | 97.67% | 98.61% | 96.98% |
| 4 weeks | 94.32% | 97.45% | 94.79% |
| 12 weeks | 50.39% | 92.83% | 89.76% |

### c) Stability of blends of fesoterodine with sugar alcohols

Blends of fesoterodine hydrogen fumarate with xylitol, sorbitol, mannitol and maltitol were stored for 6 weeks and 3 months, respectively, in closed vials at 25°C and 60% r.H., in closed vials at 40°C and 75% r.H., or in open vials at 40°C and 75% r.H. and then tested for stability by measuring the portion of undegraded fesoterodine. The initial portion of undegraded of the fesoterodine in the blends was 99.7%.

Table 5a shows that while fesoterodine is stabilized against degradation in open vials when mixed with xylitol or sorbitol, it decomposes more rapidly when mixed with mannitol and maltitol.

**Table 5a: Undegraded fesoterodine in blends after 6 weeks and 3 months, respectively**

| Blend of fesoterodine with: | closed vial 25°C, 60% r.H. | open vial 40°C, 75% r.H. |
|---|---|---|
| xylitol 1:9 | | |
| 6 weeks | 99.6 | 98.7 |
| 3 months | 99.6 | 98.2 |
| Xylitol 1:1 | | |
| 6 weeks | 99.6 | 98.6 |
| 3 months | 99.6 | 65.2 |
| Sorbitol 1:9 | | |
| 6 weeks | 99.4 | 99.0 |
| 3 months | 99.0 | 98.5 |
| Sorbitol 1:1 | | |
| 6 weeks | 99.6 | 98.7 |
| 3 months | 99.5 | 70.2 |
| Mannitol 1:9 | | |
| 6 weeks | 99.6 | 91.7 |
| 3 months | 99.4 | 42.8 |
| Mannitol 1:1 | | |
| 6 weeks | 99.7 | 94.5 |
| 3 months | 99.6 | 41.3 |
| Maltitol 1:9 | | |
| 6 weeks | 99.6 | 95.4 |
| 3 months | 99.5 | 53.7 |
| Maltitol 1:1 | | |
| 6 weeks | 99.6 | 96.3 |
| 3 months | 99.6 | 51.2 |
| Fesoterodine | | |
| 6 weeks | 99.6 | 96.3 |
| 3 months | 99.7 | 53.1 |

Table 5b shows that lactose destabilizes fesoterodine, while xylitol is capable of reducing the destabilizing effect of lactose.

**Table 5b: Undegraded fesoterodine in blends after 6 weeks and 3 months, respectively**

| Blend of fesoterodine with: | closed vial 25°C, 60% r.H. | open vial 40°C, 75% r.H. |
|---|---|---|
| Lactose 1:9 | | |
| 6 weeks | 99.7 | 86.2 |
| 3 months | 99.6 | 42.8 |
| Lactose 1:1 | | |
| 6 weeks | 99.6 | 96.2 |
| 3 months | 99.5 | 59.8 |
| Lactose/Xylitol | | |
| 1:20:9 | | |
| 6 weeks | 99.9 | 98.8 |
| 3 months | 99.6 | 97.8 |
| Lactose/Xylitol | | |
| 1:1:1 | | |
| 6 weeks | 99.6 | 98.6 |
| 3 months | 99.5 | 83.8 |

### d) Degradation of fesoterodine during granulate production

Granulates of fesoterodine with various sugars, sugar alcohols, polyols, or derivatives thereof were produced as described in Example 1. After production of granulates, and after six weeks and 3 months of storage, respectively, under various conditions, the degradation of the fesoterodine in the granulates was determined. The results are given in Table 6.

**Table 6: Degradation of fesoterodine in different granulates**

| | Closed vial 25°C, 60% r.H | | Closed vial 40°C, 75% r.H | | Open vial 40°C, 75% r.H | |
|---|---|---|---|---|---|---|
| Feso-Granulate with | %Hydr | %Degr | %Hydr | %Degr | %Hydr | %Degr |
| Mannitol 1:9 | | | | | | |
| Start | 0.45 | 0.87 | 0.45 | 0.87 | 0.45 | 0.87 |
| 6 weeks | 6.08 | 8.03 | 10.40 | 18.86 | 28.14 | 40.72 |
| 3 months | 10.37 | 12.83 | 28.15 | 57.64 | 40.52 | 63.62 |
| Maltitol 1:9 | | | | | | |
| Start | 0.42 | 0.83 | 0.42 | 0.83 | 0.42 | 0.83 |
| 6 weeks | 3.80 | 5.43 | 7.90 | 16.77 | 22.14 | 45.52 |
| 3 months | 13.85 | 17.71 | 11.44 | 29.72 | 43.11 | 72.05 |
| Xylitol 1:9 | | | | | | |
| Start | 0.06 | 0.06 | 0.06 | 0.06 | 0.06 | 0.06 |
| 6 weeks | 0.12 | 0.22 | 0.23 | 0.37 | 0.58 | 1.25 |
| 3 months | 0.23 | 0.28 | 7.04 | 14.77 | 1.14 | 4.01 |
| Sorbitol 1:9 | | | | | | |
| Start | 0.07 | 0.07 | 0.07 | 0.07 | 0.07 | 0.07 |
| 6 weeks | 0.36 | 0.51 | 1.47 | 2.79 | 0.48 | 0.91 |
| 3 months | 0.74 | 0.99 | 7.58 | 18.03 | 0.91 | 3.38 |
| Lactose 1:9 | | | | | | |
| Start | 0.35 | 0.63 | 0.35 | 0.63 | 0.35 | 0.63 |
| 6 weeks | nd | nd | 19.67 | 46.23 | 20.69 | 44.73 |
| 3 months | 20.90 | 27.90 | 17.32 | 92.55 | 21.14 | 88.25 |
| Avicel 1:9 | | | | | | |
| Start | 0.19 | 0.55 | 0.19 | 0.55 | 0.19 | 0.55 |
| 6 weeks | nd | nd | 8.47 | 13.31 | 23.57 | 55.14 |
| 3 months | 5.43 | 6.92 | 10.65 | 19.81 | 20.78 | 64.40 |
| Dextrose monohydr 1:9 | | | | | | |
| 3 weeks | 0.36* | 0.83* | 0.73 | 1.3 | | |
| 6 weeks | 0.39* | 0.81* | 1.3 | 2.3 | nd | nd |
| Isomalt 1:9 | | | | | | |
| 3 weeks | 0.39* | 0.79* | 0.75 | 1.3 | | |
| 6 weeks | 0.73* | 1.2* | 1.0 | 1.8 | nd | nd |
| Polydextrose 1:9 | | | | | | |
| 3 weeks | 0.21* | 0.58* | 0.41 | 0.8 | | |
| 6 weeks | 0.25* | 0.71* | 0.42 | 0.9 | nd | nd |

| | | | | | | |
|---|---|---|---|---|---|---|
| %Hydr = Hydrolysation product of fesoterodine (active metabolite) [% by weight] %Degr = Total degradation products of fesoterodine [% by weight]; nd=not determined * = measured at 30°C, 65% r.H. | | | | | | |

### e) Degradation of fesoterodine during tablet production

Granulates of fesoterodine with various sugars and sugar alcohols were produced as described in Example 1. These granulates were then used to produce 4 mg tablets according to Example 2.1 and with the composition given as "Example C" in Table 1, as well as wherein the xylitol was substituted with mannitol, maltitol, sorbitol, or lactose. After production of the tablets, and after six weeks and 3 months of storage, respectively, under various conditions, the amount of degradation of the fesoterodine was determined by measuring the undegraded portion of the fesoterodine. The results are shown in Table 7:

**Table 7: Degradation of fesoterodine in tablets with various sugar and sugar alcohol granulates**

| Tablets Fesoter. with: | Closed vial 25°C, 60% r.H 6 weeks | | Closed vial 40°C, 75% r.H 6 weeks | | Open vial 40°C, 75% r.H 6 weeks | |
|---|---|---|---|---|---|---|
| | %Hydr | %Degr | %Hydr | %Degr | %Hydr | %Degr |
| Mannitol 1:9 | | | | | | |
| Start | 1.23 | 1.95 | 1.23 | 1.95 | 1.23 | 1.95 |
| 6 weeks | 6.29 | 8.61 | 14.68 | 29.32 | 22.24 | 42.84 |
| 3 months | 16.58 | 23.21 | 24.06 | 60.11 | 26.62 | 69.83 |
| Maltitol 1:9 | | | | | | |
| Start | 1.11 | 1.78 | 1.11 | 1.78 | 1.11 | 1.78 |
| 6 weeks | 5.31 | 7.68 | 11.46 | 23.67 | 22.38 | 40.38 |
| 3 months | 11.16 | 15.95 | 26.87 | 68.52 | 26.65 | 69.94 |
| Xylitol 1:9 | | | | | | |
| Start | 0.06 | 0.12 | 0.06 | 0.12 | 0.06 | 0.12 |
| 6 weeks | 0.18 | 0.31 | 1.09 | 1.54 | 4.40 | 6.78 |
| 3 months | 0.64 | 0.88 | 3.91 | 6.68 | 6.76 | 13.51 |
| Sorbitol 1:9 | | | | | | |
| Start | 0.11 | 0.21 | 0.11 | 0.21 | 0.11 | 0.21 |
| 6 weeks | 0.39 | 0.64 | 2.14 | 3.66 | 6.72 | 10.48 |
| 3 months | 1.03 | 1.29 | 9.81 | 18.54 | 10.79 | 20.49 |
| Lactose 1:9 | | | | | | |
| Start | 0.67 | 1.03 | 0.67 | 1.03 | 0.67 | 1.03 |
| 6 weeks | 3.94 | 5.47 | 22.96 | 41.54 | 23.56 | 43.39 |
| 3 months | 11.14 | 15.24 | 41.54 | 51.14 | 42.74 | 51.17 |

| | | | | | | |
|---|---|---|---|---|---|---|
| %Hydr.= Hydrolysation product of fesoterodine (active metabolite) [% by weight] %Degr.= Total degradation products of fesoterodine [% by weight] | | | | | | |

### f) Stability of fesoterodine in tablets after wet granulation vs. direct compression

Tablets with the composition of "Example C" of Table 1 were obtained either (a) by the process according to Examples 1 and 2.1., i.e., by wet granulating fesoterodine and xylitol or (b) by the direct compression of all excipients. The tablets were then subjected to stability testing for 6 weeks and 3 months, respectively, at 40°C, 75% r.H. in closed vials. The results are shown in Table 8.

**Table 8: Comparison of fesoterodine 6-week and 3 months-stress stability, respectively, in tablets after (a) wet granulation or (b) direct compression in the presence of xylitol**

| Tablets produced by | % Hydrolysation by weight | % Total Degradation by weight |
|---|---|---|
| wet granulation | | |
| 6 weeks | 0.48 | 0.68 |
| 3 months | 1.69 | 2.92 |
| direct compression | | |
| 6 weeks | 1.32 | 2.08 |
| 3 months | 4.71 | 8.72 |

| | | |
|---|---|---|
| Hydrolyzation by weight refers to the percent by weight of active metabolite. Total degradation by weight refers to the percent by weight of total degradation products of fesoterodine. | | |

### g) Stability of fesoterodine in tablets after dry granulation

Tablets with the composition of "Example C" of Table 1 were prepared as follows:
3.5 kg fesoterodine hydrogen fumarate was blended with 31.5 kg xylitol and sieved through a 0.032" sieve. The blend was dry compacted with a roller compactor at a pressure of 1250 psi.

The compacted ribbons were granulated via a 20 mesh screen in an oscillator granulator. The material was further screened through a 0.040" grater screen. 33.91 MICROCELAC® 100 and 15.09 kg hypromellose K100M were added and blended. After sieving over 0.040", the blend was dry compacted at 700 psi. After compaction, the material was granulated via a 16 mesh oscillating sieve and finally via a 0.062" grater screen. 37.41 kg hypromellose K100 M, 0.5 kg hypromellose K4M and 3.72 kg talc were added, blended and the blend sieved via a 0.062" grater screen. After subsequent blending, 4.38 kg glyceryl behenate were added and the mixture was blended to the press mixture. The finished press mixture was transferred to a rotary tablet machine and compressed to oval biconvex tablets.

OPADRY® and purified water were added to a vessel under stirring. The mixture was stirred for at least 1 h. Then the suspension was passed through a sieve of a suitable size (e.g., 300 µm) and transferred into the solution tank of the coating pan (e.g., driacoater 1200). A film coating was applied on the cores under constant stirring of the suspension until the weight per coated tablet was 335 mg.

The tablets were subjected to stability testing at room temperature for 6 months in closed vials.

Table 9 shows the result of the stability testing of the composition produced by dry granulation compared to a composition produced by wet granulation according to Example 1.

**Table 9**

| Composition produced by | Hydrolyzation by weight | Total degradation by weight |
|---|---|---|
| Dry Granulation | 0.83 | 1.79 |
| Wet Granulation | 0.27 | 1.33 |

Hydrolyzation by weight refers to the percent by weight of active metabolite. Total degradation by weight refers to the percent by weight of total degradation products of fesoterodine.

### 7. In vitro dissolution profile of pharmaceutical compositions containing various amounts of HPMC

The in-vitro dissolution profiles of fesoterodine tablets with different HPMC contents were determined. The composition of the tablets is shown in Table 10 below.

**Table 10**

| Formulation number | I | J | K | L | M | N |
|---|---|---|---|---|---|---|
| Dosage | 4 mg | 4 mg | 4 mg | 8mg | 8mg | 8mg |
| | [mg] | [mg] | [mg] | [mg] | [mg] | [mg] |
| Fesoterodine/Xylitol Granulate 1:9 | 40.00 | 40.00 | 40.00 | 80,00 | 80,00 | 80,00 |
| MICROCELAC® 100 | 121.50 | 165.50 | 197.50 | 77,50 | 125,50 | 157,50 |
| METHOCEL® K100M | 70.00 | 48.00 | 32.00 | 120,00 | 80,00 | 53,00 |
| METHOCEL® K4M | 70.00 | 48.00 | 32.00 | 24,00 | 16,00 | 11,00 |
| Compritol 888 ATO | 10.00 | 10.00 | 10.00 | 10,00 | 10,00 | 10,00 |
| Talc | 8.50 | 8.50 | 8.50 | 8,50 | 8,50 | 8,50 |
| Total | 320.00 | 320.00 | 320.00 | 320,00 | 320,00 | 320,00 |

The in-vitro dissolution profiles were determined by the following analytical methods:
Drug release testing of fesoterodine SR 4mg and 8mg tablets

### Dissolution parameters

| | |
|---|---|
| Dissolution Tester: | e.g. Erweka DT800 |
| Dissolution method: | according to USP <711> Drug Release, App. 2 |
| Temperature: | 37°C ± 0.5°C |
| RPM: | 75 |
| Sampling volume: | 5 mL (medium replacement after each sampling) |
| Sinker: | yes |
| Dissolution Medium: | phosphate buffer pH 6.8 |
| | |
| Chromatographic Conditions | |
| Column: | Spherisorb CN, 5 µm, 250 mm x 4 mm |
| Mobile phase: | Acetonitrile/Water/Trifluoro acetic acid 550:450:1 (v/v/v) |
| Flow rate: | 0.8 mL/min |
| Oven temperature: | 35°C |
| Autosampler temperature: | 20°C |
| Injection volume: | 50 µL |
| Detection: | UV at 220 nm |
| Retention times: | |
| Fesoterodine hydrogen fumarate | approx. 4.4 min |
| Active metabolite | approx. 4.0 min |
| Run Time: | 6.5 min |

The dissolution results are shown in Table 11a and Table 11b below and in Figures 1 and 2.

**Table 11a**

| Batch Number | I | J | K |
|---|---|---|---|
| Relative amount of HPMC | 43.75 wt% | 30 wt% | 20wt% |
| Time in hours | % Release of fesoterodine hydrogen fumarate | | |
| 0 | 0 | 0 | 0 |
| 1 | 18 | 21 | 19 |
| 2 | 31 | 35 | 37 |
| 4 | 49 | 55 | 59 |
| 16 | 93 | 96 | 76 |

**Table 11b**

| Batch Number | L | M | N |
|---|---|---|---|
| Relative amount of HPMC | 45 wt% | 30 wt% | 20 wt% |
| Time in hours | % Release of fesoterodine hydrogen fumarate | | |
| 0 | 0 | 0 | 0 |
| 1 | 18 | 21 | 23 |
| 2 | 31 | 37 | 40 |
| 4 | 50 | 60 | 63 |
| 16 | 93 | 100 | 96 |

Numbers refer to the percent release of fesoterodine hydrogen fumarate. The total amount of fesoterodine hydrogen fumarate in the tablets was 4 mg.

Based on these dissolution profiles the formulations having a HPMC content of over 30 % (formulations "I" and "L") are particularly preferred. Another formulation matching the most preferred dissolution profile was formulation "O" given in Table 11c below:

**Table 11c**

| **Formulation O** | |
|---|---|
| Tablet Core | |
| Fesoterodine hydrogen fumarate | 4.0 |
| Xylitol | 76.0 |
| Lactose monohydrate | 43.0 |
| Microcrystalline cellulose | 41.5 |
| Hypromellose (e.g. METHOCEL® K100M) | 70.0 |
| Hypromellose (e.g. METHOCEL® K4M) | 70.0 |
| Glycerol dibehenate | 8.0 |
| Talc | 7.5 |
| Purified water | q.s.* |
| Film-coat | - |
| | |
| Total | 330.0 |

### 8. Comparison of the efficacy, tolerability, and safety of fesoterodine hydrogen fumarate and tolterodine formulations

A randomized, double-blind, double dummy, placebo and active-controlled trial in adult male and female human subjects was conducted. Once-daily 4 mg and 8 mg fesoterodine hydrogen fumarate was orally administered to patients with overactive bladder and compared to placebo and active-control (4 mg/day tolterodine ER (extended release)).

The fesoterodine formulations were as follows:
4 mg: Example A according to Table 1 herein
8 mg: Examples F and G according to Table 2 herein

The trial was conducted in 19 countries. A total of 1135 subjects were randomized and 1103 subjects were included in the full analysis set: 279 in the placebo treatment group, 265 in the 4 mg fesoterodine treatment group, 276 in the 8 mg fesoterodine treatment group, and 283 in the 4 mg tolterodine ER treatment group. The mean age of subjects was 57 years. A total of 81% were female and 97% were white. There were no notable differences among the treatment groups with respect to gender, race, age, or weight/BMI.

The efficacy results demonstrated statistically significant (all p ≤ 0.001) improvements compared to placebo at the end of treatment in the primary variables requested by both the USFDA (change in average number of micturitions per 24 hours and change in the average number of urge incontinence episodes per 24 hours) and European regulatory authorities (change in average.number of micturitions per 24 hours and treatment response (Yes/No variable) derived from a 4-category Treatment Benefit Scale). The decreases in the number of micturitions and urge incontinence episodes per 24 hours in the fesoterodine 8 mg/day and 4 mg/day groups were significantly higher than in the placebo group. The treatment response rates also were higher at the end of treatment in both fesoterodine dose groups compared to placebo. Tolterodine ER showed statistically significant (all p ≤ 0.008) improvements compared to placebo at the end of treatment for the above mentioned primary variables. For all primary variables, fesoterodine 4 mg/day demonstrated a slightly higher numerical change compared to placebo than tolterodine ER. This numerical difference was more pronounced for fesoterodine 8 mg/day.

A total of 1132 subjects were included in the safety set: 283 in the placebo treatment group, 272 in the fesoterodine 4 mg/day treatment group, 287 in the fesoterodine 8 mg/day treatment group, and 290 in the tolterodine ER 4 mg/day treatment group. The most common adverse events were typical of those observed in subjects taking marketed antimuscarinics. Dry mouth occurred more frequently in fesoterodine-treated and tolterodine-treated subjects compared to placebo-treated subjects (Fesoterodine 4 mg/day 22%, fesoterodine 8 mg/day 34%, tolterodine 4 mg/day 17%, placebo 7%). Constipation occurred more frequently in fesoterodine-treated and tolterodine-treated subjects compared to placebo-treated subjects (Fesoterodine 4 mg/day 3%, fesoterodine 8 mg/day 5%, tolterodine 4 mg/day 3%, placebo 1%). Keratoconjunctivitis sicca occurred more frequently in fesoterodine-treated and tolterodine-treated subjects compared to placebo-treated subjects (Fesoterodine 4 mg/day 2%, fesoterodine 8 mg/day 4%, tolterodine 4 mg/day 1%, placebo 0%). The incidence of dry throat was low in all groups ((Fesoterodine 4 mg/day 1%, fesoterodine 8 mg/day 3%, tolterodine 4 mg/day 1%, placebo 0%) as was urinary retention (Fesoterodine 4 mg/day 1%, fesoterodine 8 mg/day 1%, tolterodine 4 mg/day 0%, placebo 0%) and blurred vision (Fesoterodine 4 mg/day 1%, fesoterodine 8 mg/day 1%, tolterodine 4 mg/day 1%, placebo 2%).

A summary of the results of the closed testing procedure is provided in Table 12.

**Table 12**

| **Summary of statistical analyses of primary efficacy variables using closed testing procedure (FAS with LOCF in SP583)** | | |
|---|---|---|
| **Testing procedure** | **p-value^{a}** | **Significant^{b}** |
| **United States Food and Drug Administration** | | |
| Step 1: Number of micturitions per 24 hours | <0.001 | Yes |
| Feso 8mg/day vs pbo | | |
| Step 2: Number of micturitions per 24 hours | <0.001 | Yes |
| Feso 4mg/day vs pbo | | |
| Step 3: Number of urge incontinence episodes per 24 hours | <0.001 | Yes |
| Feso 8mg/day vs pbo | | |
| Step. 4: Number of urge incontinence episodes per 24 hours | 0.001 | Yes |
| Feso 4mg/day vs pbo | | |

| **European Regulatory Authorities** | **p-value^{a}** | **Significant^{c}** |
|---|---|---|
| Step 1: Feso 8mg/day vs pbo | | Yes |
| a) Number of micturitions per 24 hours | <0.001 | |
| b) Treatment response | <0.001 | |
| Step 2: Feso 4mg/day vs pbo | | Yes |
| a) Number of micturitions per 24 hours | <0-001 | |
| b) Treatment response | <0.001 | |

| | | |
|---|---|---|
| FAS-full analysis set, LOCF=last observation carried forward a Number of micturitions and urge incontinence episodes analysed with an ANCOVA model with terms for treatment and region and baseline value as a covariate. Treatment response is analyzed using a normal approximation method for binary data. b Significant based on closed testing procedure. Each step tested at 0.05 two-sided (0.025 one-sided). If result was not significant at any step, then all steps after that are considered not statistically significant c Significant based on closed testing procedure. If both p-values at Step 1 are less than 0.05 two-sided (0.025 one-sided), then 8mg results are statistically significant. If Step 1 is statistically significant and both p-values at Step 2 are less than 0.05 two-sided (0.025 one-sided), thcn 4mg results are statistically significant NOTE: All results are at the end of treatment using LOCF for missing values | | |

For the USFDA analysis, all 4 p-values in the closed testing procedure were less than 0.05. Therefore, both fesoterodine doses (4 mg and 8 mg/day) showed statistically significant improvement over placebo at end of treatment for the micturitions and urge incontinence variables.

Similarly, for the European regulatory authorities analysis, all 4 p-values in the closed testing procedure were less than 0.05. Therefore, both fesoterodine doses (4 mg and 8 mg/day) showed statistically significant improvement over placebo at end of treatment for the micturitions and treatment response variables.

## Claims

1. A pharmaceutical composition comprising fesoterodine, or a pharmaceutically acceptable salt or solvate thereof, and a pharmaceutically acceptable stabilizer, wherein said stabilizer is selected from the group consisting of xylitol, sorbitol, polydextrose, isomalt, dextrose and combinations thereof.

2. The pharmaceutical composition according to claim 1, wherein said stabilizer is xylitol, sorbitol or polydextrose.

3. The pharmaceutical composition according to claim 1, wherein said stabilizer is xylitol.

4. A pharmaceutical composition according to any one of the preceding claims, wherein the fesoterodine/stabilizer ratio is 1%-20% [w/w].

5. A pharmaceutical composition according to any one of the preceding claims, which comprises a salt of fesoterodine which has an auto pH in water of 3-5.

6. A pharmaceutical composition according to claim 5, wherein the fesoterodine salt is a salt of a di- or tricarboxylic acid, or of a partially hydrogenated di- or tricarboxylic acid.

7. A pharmaceutical composition according to any one of the preceding claims, which comprises fesoterodine hydrogen fumarate.

8. A pharmaceutical composition according to claim 7, which is in unit dosage form and **characterized in that** fesoterodine hydrogen fumarate is present in an amount of between 0.5 and 12 mg per dosage unit.

9. A pharmaceutical composition according to anyone of the preceding claims, which is obtainable by a method involving at least one granulation step.

10. A pharmaceutical composition according to claim 9, wherein the granulation is wet granulation.

11. A pharmaceutical composition according to claim 10, wherein the granulation is performed in the presence of water.

12. A pharmaceutical composition according to any one of the preceding claims, wherein the pharmaceutical composition further comprises a sustained release agent.

13. A pharmaceutical composition according to claim 12, wherein the sustained release agent is a cellulose ether or ester or a mixture thereof.

14. A pharmaceutical composition according to claim 13, wherein the sustained release agent is hydroxypropylmethylcellulose.

15. A pharmaceutical composition according to any one of claims 12 to 14, wherein the sustained release agent is contained in an amount of 20-80% [w/w], preferably 25-65%, more preferably 30-65%, and even more preferably 35-55% [w/w] based on the total composition.

16. A pharmaceutical composition according to any one of claims 12 to 15, which exhibits a cumulated fesoterodine release (in weight percent based on the theoretical amount of fesoterodine in the formulation) in an in vitro dissolution assay according to USP 711 (in phosphate buffer pH 6.8, 37°C, at 75 rpm) as follows:
• 5% to 30%, preferably 6% to 26% fesoterodine release after 1 hour,
• 15% to 40%, preferably 18% to 38% fesoterodine release after 2 hours,
• 35% to 65%, preferably 36% to 56% fesoterodine release after 4 hours, and
• at least 75%, preferably at least 80% fesoterodine release after 16 hours.

17. A pharmaceutical composition according to claim 1 comprising:
(a) 0.3-5.0% [w/w] of fesoterodine hydrogen fumarate;
(b) 5-25% [w/w] sorbitol or xylitol;
(c) 20-40% [w/w] of a mixture comprising 45-80% (w/w) lactose monohydrate and 55-20% (w/w) microcrystalline cellulose;
(d) 20-65% hydroxypropyl methylcellulose;
(e) 1-5% [w/w] glycerol dibehenate; and
(f) 1-5% [w/w] talc.

18. The pharmaceutical composition of claim 17, comprising
(a) 4.0 mg fesoterodine hydrogen fumarate
(b) 32-40 mg of Xylitol with a mean particle size of about 0.001-0.30 mm)
(c) 115-130 mg of MICROCELAC^{®} 100
(d) 65-75 mg of HPMC having a nominal viscosity of about 100,000 mPa·s when dissolved (about 2% by weight) in water
(e) 65-75 mg of HPMC having a nominal viscosity of about 4,000 mPa·s when dissolved about 2% by weight) in water
(f) 8-12 mg of Glycerol dibehenate
(g) 7-10 mg Talc, and, optionally,
(h) a coating.

19. The pharmaceutical composition of claim 17, comprising
(a) 8.0 mg fesoterodine hydrogen fumarate
(b) 65-80 mg of Xylitol with a mean particle size of about 0.001-0.30 mm)
(c) 70-85 mg of MICROCELAC^{®} 100
(d) 110-130 mg of HPMC having a nominal viscosity of about 100,000 mPa·s when dissolved (about 2% by weight) in water
(e) 20-30 mg of HPMC having a nominal viscosity of about 4,000 mPa·s when dissolved about 2% by weight) in water
(f) 8-12 mg of Glycerol dibehenate
(g) 7-10 mg Talc, and, optionally,
(h) a coating.

20. The pharmaceutical composition according to any one of the preceding claims for use in treating overactive bladder.

21. The pharmaceutical composition for use according to claim 20, wherein overactive bladder is associated with symptoms selected from the group consisting of urinary incontinence, urinary urge incontinence, imperative urinary urge, and/or increased urinary frequency.

22. Use of a substance selected from the group consisting of xylitol, sorbitol, polydextrose, isomalt, and dextrose for the stabilization of a pharmaceutical composition comprising fesoterodine, or a pharmaceutically acceptable salt or solvate thereof.

23. A method for preparing a pharmaceutical composition according to any of claims 1-19, comprising mixing of Fesoterodine or a pharmaceutically acceptable salt or solvate thereof with a stabilizer selected from the group consisting of xylitol, sorbitol, polydextrose, isomalt, dextrose and combinations thereof.

24. The method according to claim 23 which further comprises a granulation step.

25. The method according to claim 24 wherein the granulation is wet granulation.

26. The method according to anyone of claims 23-25 comprising
• granulating Fesoterodine or a pharmaceutically acceptable salt or solvate thereof with a stabilizer selected from the group consisting of xylitol, sorbitol, polydextrose, isomalt, dextrose and combinations thereof in the presence of water,
• drying the granulate,
• mixing the dried granulates with at least one other excipient to give a compression mixture,
• compressing the compression mixture to the desired form, and
• optionally applying a coating.

## Patentansprüche

1. Pharmazeutische Zusammensetzung, die Fesoterodin oder ein pharmazeutisch annehmbares Salz oder Solvat davon und einen pharmazeutisch annehmbaren Stabilisator umfasst, wobei der Stabilisator aus der Gruppe bestehend aus Xylit, Sorbitol, Polydextrose, Isomalt, Dextrose und Kombinationen davon ausgewählt ist.

2. Pharmazeutische Zusammensetzung gemäss Anspruch 1, wobei der Stabilisator Xylit, Sorbitol oder Polydextrose ist.

3. Pharmazeutische Zusammensetzung gemäss Anspruch 1, wobei der Stabilisator Xylit ist.

4. Pharmazeutische Zusammensetzung gemäss einem der vorhergehenden Ansprüche, wobei der Fesoterodin/Stabilisator-Anteil 1 bis 20 % (G/G) beträgt.

5. Pharmazeutische Zusammensetzung gemäss einem der vorhergehenden Ansprüche, die ein Fesoterodinsalz umfasst, das in Wasser einen Auto-pH von 3 bis 5 aufweist.

6. Pharmazeutische Zusammensetzung gemäss Anspruch 5, wobei das Fesoterodinsalz ein Salz einer Di- oder Tricarbonsäure oder einer teilhydrierten Di- oder Tricarbonsäure ist.

7. Pharmazeutische Zusammensetzung gemäss einem der vorhergehenden Ansprüche, welche Fesoterodinhydrogenfumarat umfasst.

8. Pharmazeutische Zusammensetzung gemäss Anspruch 7, die in Einzeldosierungsform vorliegt und **dadurch gekennzeichnet ist, dass** Fesoterodinhydrogenfumarat in einer Menge zwischen 0,5 und 12 mg pro Dosierungseinheit vorliegt.

9. Pharmazeutische Zusammensetzung gemäss einem der vorhergehenden Ansprüche, die durch ein Verfahren erhältlich ist, das mindestens einen Granulierungsschritt umfasst.

10. Pharmazeutische Zusammensetzung gemäss Anspruch 9, wobei die Granulierung eine Nassgranulierung ist.

11. Pharmazeutische Zusammensetzung gemäss Anspruch 10, wobei die Granulierung in der Gegenwart von Wasser durchgeführt wird.

12. Pharmazeutische Zusammensetzung gemäss einem der vorhergehenden Ansprüche, wobei die pharmazeutische Zusammensetzung ferner ein Retardierungsmittel umfasst.

13. Pharmazeutische Zusammensetzung gemäss Anspruch 12, wobei das Retardierungsmittel ein Celluloseether oder -ester oder ein Gemisch davon ist.

14. Pharmazeutische Zusammensetzung gemäss Anspruch 13, wobei das Retardierungsmittel Hydroxypropylmethylcellulose ist.

15. Pharmazeutische Zusammensetzung gemäss einem der Ansprüche 12 bis 14, wobei das Retardierungsmittel in einer Menge von 20 bis 80 % (G/G), bevorzugt 25 bis 65 %, stärker bevorzugt 30 bis 65 % und besonders bevorzugt 35 bis 55 % (G/G), bezogen auf die gesamte Zusammensetzung, enthalten ist.

16. Pharmazeutische Zusammensetzung gemäss einem der Ansprüche 12 bis 15, welche die folgende kumulative Fesoterodin-Freisetzung (in Gew.%, bezogen auf die theoretische Menge an Fesoterodin in der Formulierung) in einem in vitro-Dissolution-Test gemäss USP 711 (in Phosphatpuffer pH 6,8, 37°C, bei 75 U/min) aufweist:
• 5 bis 30 %, vorzugsweise 6 bis 26 %, Fesoterodin-Freisetzung nach 1 Stunde,
• 15 bis 40 %, vorzugsweise 18 bis 38 %, Fesoterodin-Freisetzung nach 2 Stunden,
• 35 bis 65 %, vorzugsweise 36 bis 56 %, Fesoterodin-Freisetzung nach 4 Stunden und
• mindestens 75 %, vorzugsweise mindestens 80 %, Fesoterodin-Freisetzung nach 16 Stunden.

17. Pharmazeutische Zusammensetzung gemäss Anspruch 1, umfassend:
(a) 0,3 bis 5,0 % (G/G) Fesoterodinhydrogenfumarat;
(b) 5 bis 25 % (G/G) Sorbitol oder Xylit;
(c) 20 bis 40 % (G/G) einer Mischung, die 45 bis 80 % (G/G) Lactosemonohydrat und 55 bis 20 % (G/G) mikrokristalline Cellulose umfasst;
(d) 20 bis 65 % Hydroxypropylmethylcellulose;
(e) 1 bis 5 % (G/G) Glycerindibehenat und
(f) 1 bis 5 % (G/G) Talk.

18. Pharmazeutische Zusammensetzung gemäss Anspruch 17, umfassend:
(a) 4,0 mg Fesoterodinhydrogenfumarat;
(b) 32 bis 40 mg Xylit mit einer durchschnittlichen Partikelgrösse von etwa 0,001 bis 0,30 mm);
(c) 115 bis 130 mg MICROCELAC^{®} 100;
(d) 65 bis 75 mg HPMC, die eine nominale Viskosität von etwa 100.000 mPa·s besitzt, wenn sie in Wasser (etwa 2 Gew.%) gelöst ist;
(e) 65 bis 75 mg HPMC, die eine nominale Viskosität von etwa 4.000 mPa·s besitzt, wenn sie in Wasser (etwa 2 Gew.%) gelöst ist;
(f) 8 bis 12 mg Glycerindibehenat;
(g) 7 bis 10 mg Talk und, wahlweise,
(h) einen Überzug.

19. Pharmazeutische Zusammensetzung gemäss Anspruch 17, umfassend:
(a) 8,0 mg Fesoterodinhydrogenfumarat;
(b) 65 bis 80 mg Xylit mit einer durchschnittlichen Partikelgrösse von etwa 0,001 bis 0,30 mm);
(c) 70 bis 85 mg MICROCELAC^{®} 100;
(d) 110 bis 130 mg HPMC, die eine nominale Viskosität von etwa 100.000 mPa·s besitzt, wenn sie in Wasser (etwa 2 Gew.%) gelöst ist;
(e) 20 bis 30 mg HPMC, die eine nominale Viskosität von etwa 4.000 mPa·s besitzt, wenn sie in Wasser (etwa 2 Ges.%) gelöst ist;
(f) 8 bis 12 mg Glycerindibehenat;
(g) 7 bis 10 mg Talk und, wahlweise,
(h) einen Überzug.

20. Pharmazeutische Zusammensetzung gemäss einem der vorhergehenden Ansprüche zur Verwendung bei der Behandlung einer hyperaktiven Blase.

21. Pharmazeutische Zusammensetzung zur Verwendung gemäss Anspruch 20, wobei die hyperaktive Blase mit Symptomen, ausgewählt aus der Gruppe bestehend aus Harninkontinenz, Harndranginkontinenz, zwingendem Harndrang und/oder erhöhter Häufigkeit des Harnlassens, verbunden ist.

22. Verwendung einer Substanz, ausgewählt aus der Gruppe bestehend aus Xylit, Sorbitol, Polydextrose, Isomalt und Dextrose, zur Stabilisierung einer pharmazeutischen Zusammensetzung, die Fesoterodin oder ein pharmazeutisch annehmbares Salz oder Solvat davon umfasst.

23. Verfahren zur Herstellung einer pharmazeutischen Zusammensetzung gemäss einem der Ansprüche 1 bis 19, die das Mischen von Fesoterodin oder einem pharmazeutisch annehmbaren Salz oder Solvat davon mit einem Stabilisator, ausgewählt aus der Gruppe bestehend aus Xylit, Sorbitol, Polydextrose, Isomalt, Dextrose und Kombinationen davon, umfasst.

24. Verfahren gemäss Anspruch 23, das ferner einen Granulierungsschritt umfasst.

25. Verfahren gemäss Anspruch 24, wobei die Granulierung eine Nassgranulierung ist.

26. Verfahren gemäss einem der Ansprüche 23 bis 25, umfassend:
• das Granulieren von Fesoterodin oder einem pharmazeutisch annehmbaren Salz oder Solvat davon mit einem Stabilisator, ausgewählt aus der Gruppe bestehend aus Xylit, Sorbitol, Polydextrose, Isomalt, Dextrose und Kombinationen davon, in der Gegenwart von Wasser,
• das Trocknen des Granulats,
• das Mischen des getrockneten Granulats mit mindestens einem weiteren Hilfsstoff, um eine Tablettierungsmischung zu erhalten;
• das Verpressen der Tablettierungsmischung in die gewünschte Form und
• das wahlweise Aufbringen eines Überzugs.

## Revendications

1. Composition pharmaceutique comprenant de la fésotérodine, ou un sel ou un solvate de celle-ci pharmaceutiquement acceptable, et un stabilisant pharmaceutiquement acceptable, dans laquelle ledit stabilisant est choisi dans le groupe constitué du xylitol, du sorbitol, du polydextrose, de l'isomalt, du dextrose et de combinaisons de ceux-ci.

2. Composition pharmaceutique selon la revendication 1, dans laquelle ledit stabilisant est le xylitol, le sorbitol ou le polydextrose.

3. Composition pharmaceutique selon la revendication 1, dans laquelle ledit stabilisant est le xylitol.

4. Composition pharmaceutique selon l'une quelconque des revendications précédentes, dans laquelle le rapport fésotérodine/stabilisant est de 1 % à 20 % (en poids).

5. Composition pharmaceutique selon l'une quelconque des revendications précédentes, qui comprend un sel de fésotérodine qui possède un auto pH dans l'eau de 3 à 5.

6. Composition pharmaceutique selon la revendication 5, dans laquelle le sel de fésotérodine est un sel d'un acide di- ou tri-carboxylique, ou d'un acide di- ou tri-carboxylique partiellement hydrogéné.

7. Composition pharmaceutique selon l'une quelconque des revendications précédentes, qui comprend de l'hydrogénofumarate de fésotérodine.

8. Composition pharmaceutique selon la revendication 7, qui se présente sous forme de dose unitaire, et ***caractérisée en ce que*** l'hydrogénofumarate de fésotérodine est présent selon une quantité comprise entre 0,5 et 12 mg par unité de prise.

9. Composition pharmaceutique selon l'une quelconque des revendications précédentes, qui peut être obtenue par un procédé impliquant au moins une étape de granulation.

10. Composition pharmaceutique selon la revendication 9, dans laquelle la granulation est une granulation par voie humide.

11. Composition pharmaceutique selon la revendication 10, dans laquelle la granulation est réalisée en présence d'eau.

12. Composition pharmaceutique selon l'une quelconque des revendications précédentes, dans laquelle la composition pharmaceutique comprend en outre un agent permettant une libération prolongée.

13. Composition pharmaceutique selon la revendication 12, dans laquelle l'agent permettant la libération prolongée est un éther ou un ester de cellulose ou un mélange de ceux-ci.

14. Composition pharmaceutique selon la revendication 13, dans laquelle l'agent permettant la libération prolongée est l'hydroxypropylméthylcellulose.

15. Composition pharmaceutique selon l'une quelconque des revendications 12 à 14, dans laquelle l'agent permettant la libération prolongée est contenu selon une quantité de 20 à 80 % (en poids), préférablement de 25 à 65 %, plus préférablement de 30 à 65 %, et de manière encore davantage préférée de 35 à 55 % (en poids) sur la base de la composition totale.

16. Composition pharmaceutique selon l'une quelconque des revendications 12 à 15. qui affiche une libération de fésotérodine cumulée (en pourcentage pondéral sur la base de la quantité théorique de fésotérodine dans la formulation) dans un test de dissolution in vitro selon l'USP 711 (dans un tampon phosphaté, pH 6.8, 37°C, à 75 tr/min) comme suit :
• libération de fésotérodine après 1 heure : 5 % à 30 %, préférablement 6 % à 26 %,
• libération de fésotérodine après 2 heures : 15 % à 40 %, préférablement 18 % à 38 %,
• libération de fésotérodine après 4 heures : 35 % à 65 %, préférablement 36 % à 56 %, et
• libération de fésotérodine après 16 heures : au moins 75 %, préférablement au moins 80%.

17. Composition pharmaceutique selon la revendication 1, comprenant :
(a) 0,3 à 5,0 % (en poids) d'hydrogénofumarate de fésotérodine ;
(b) 5 à 25 % (en poids) de sorbitol ou de xylitol ;
(c) 20 à 40 % (en poids) d'un mélange comprenant 45 à 80 % (en poids) de monohydrate de lactose et 55 à 20 % (en poids) de cellulose microcristalline ;
(d) 20 à 65 % d'hydroxypropyl méthylcellulose ;
(e) 1 à 5 % (en poids) de dibéhénate de glycérol ; et
(f) 1 à 5 % (en poids) de talc.

18. Composition pharmaceutique selon la revendication 17. comprenant :
(a) 4 mg d'hydrogénofumarate de fésotérodine
(b) 32 à 40 mg de xylitol avec une taille moyenne des particules d'environ 0,001 à 0,30 mm
(c) 115 à 130 mg de MICROCELAC^{©} 100
(d) 65 à 75 mg d'HMPC (hydroxypropyl méthylcellulose) ayant une viscosité nominale d'environ 100 000 mPa·s lorsqu'elle est dissoute (environ 2 % en poids) dans de l'eau
(e) 65 à 75 mg d'HMPC ayant une viscosité nominale d'environ 4 000 mPa·s lorsqu'elle est dissoute (environ 2 % en poids) dans de l'eau
(f) 8 à 12 mg de dibéhénate de glycérol
(g) 7 à 10 mg de talc et, facultativement,
(h) un enrobage.

19. Composition pharmaceutique selon la revendication 17, comprenant :
(a) 8 mg d'hydrogénofumarate de fésotérodine
(b) 65 à 80 mg de xylitol avec une taille moyenne des particules d'environ 0,001 à 0,30 mm
(c) 70 à 85 mg de MICROCELAC^{©} 100
(d) 110 à 130 mg d'HMPC ayant une viscosité nominale d'environ 100 000 mPa·s lorsqu'elle est dissoute (environ 2 % en poids) dans de l'eau
(e) 20 à 30 mg d'HMPC ayant une viscosité nominale d'environ 4 000 mPa·s lorsqu'elle est dissoute (environ 2 % en poids) dans de l'eau
(f) 8 à 12 mg de dibéhénate de glycérol
(g) 7 à 10 mg de talc et, facultativement.
(h) un enrobage.

20. Composition pharmaceutique selon l'une quelconque des revendications précédentes, destinée à un usage dans le traitement de la vessie hyperactive.

21. Composition pharmaceutique destinée à un usage selon la revendication 20, dans laquelle la vessie hyperactive est associée à des symptômes choisis dans le groupe constitué de l'incontinence urinaire, de l'incontinence avec urgent besoin d'uriner, de l'envie impérative d'uriner, et/ou une fréquence de mictions accrue.

22. Utilisation d'une substance choisie dans le groupe constitué du xylitol, du sorbitol, du polydextrose, de l'isomalt et du dextrose, pour la stabilisation d'une composition pharmaceutique comprenant de la fésotérodine, ou un sel ou un solvate pharmaceutiquement acceptable de celle-ci.

23. Procédé de préparation d'une composition pharmaceutique selon l'une quelconque des revendications 1 à 19, comprenant le mélange de fésotérodine ou d'un sel ou d'un solvate pharmaceutiquement acceptable de celle-ci avec un stabilisant choisi dans le groupe constitué du xylitol, du sorbitol, du polydextrose, de l'isomalt, du dextrose et de combinaisons de ceux-ci.

24. Procédé selon la revendication 23, qui comprend en outre une étape de granulation.

25. Procédé selon la revendication 24, dans lequel l'étape de granulation est une étape de granulation par voie humide.

26. Procédé selon l'une quelconque des revendications 23 à 25, comprenant :
• la granulation de fésotérodine ou d'un sel ou d'un solvate pharmaceutiquement acceptable de celle-ci avec un stabilisant choisi dans le groupe constitué du xylitol, du sorbitol, du polydextrose, de l'isomalt, du dextrose et de combinaisons de ceux-ci, en présence d'eau,
• le séchage du granulat,
• le mélange des granulés séchés avec au moins un autre excipient pour donner un mélange de compression,
• la compression du mélange de compression sous la forme désirée, et
• facultativement, l'application d'un enrobage.
